(19) Europäisches Patentamt
European Patent Office
Office européen des brevets

(11) **EP 4 588 936 A1**

(12) **EUROPEAN PATENT APPLICATION**
published in accordance with Art. 153(4) EPC

(43) Date of publication:
23.07.2025 Bulletin 2025/30

(21) Application number: 23900084.7

(22) Date of filing: 08.12.2023

(51) International Patent Classification (IPC):
*C07K 16/10* (2006.01)  *C12N 15/09* (2006.01)
*C12N 5/10* (2006.01)  *C12N 1/19* (2006.01)
*C12N 1/21* (2006.01)  *C12N 1/15* (2006.01)
*A61K 39/395* (2006.01)  *A61P 31/14* (2006.01)
*A61P 11/00* (2006.01)

(52) Cooperative Patent Classification (CPC):
A61K 39/395; A61K 39/42; A61P 11/00;
A61P 31/14; C07K 16/00; C07K 16/10; C12N 5/10;
C12N 15/09; C12N 15/74; C12N 15/80; C12N 15/81

(86) International application number:
PCT/CN2023/137388

(87) International publication number:
WO 2024/120516 (13.06.2024 Gazette 2024/24)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 08.12.2022  CN 202211571449
04.01.2023  CN 202310007283

(71) Applicant: Changchun Bcht Biotechnology Co.
Jilin 130012 (CN)

(72) Inventors:
• XU, Xiaoyu
Nanjing, Jiangsu 210046 (CN)
• CAO, Lin
Nanjing, Jiangsu 210046 (CN)
• WANG, Xiangxi
Beijing 100101 (CN)
• XU, Lingjie
Nanjing, Jiangsu 210046 (CN)

(74) Representative: Gill Jennings & Every LLP
The Broadgate Tower
20 Primrose Street
London EC2A 2ES (GB)

Remarks:
The complete document including Reference
Table(s) and the Sequence Listing(s) can be
downloaded from the EPO website

(54) **ANTIBODIES SPECIFICALLY BINDING TO RSV**

(57) Provided are a set of antibodies specifically binding to RSV or antigen-binding fragments thereof, as well as a corresponding polynucleotide, vector, host cell, composition comprising said antibodies, and method for generating the antibodies.

FIG. 1-1

**EP 4 588 936 A1**

**Description**

**RELATED APPLICATIONS INCORPORATED BY REFERENCE**

The present application claims the right of priority for Chinese invention patent application No. CN202211571449.4 filed on December 08, 2022 and Chinese invention patent application No. CN202310007283.1 filed on January 04, 2023, which are incorporated herein by reference in their entireties.

**TECHNICAL FIELD**

The present invention provides a set of antibodies or antigen-binding fragments thereof that specifically bind to RSV. The present invention also provides a polynucleotide encoding the antibody or the antigen-binding fragment thereof, a vector comprising the polynucleotide, a host cell comprising the vector, a method for generating the antibody, and a composition comprising the antibody.

**BACKGROUND ART**

Respiratory syncytial virus (RSV) causes about 33 million acute lower respiratory infections and over 100,000 deaths per year in children under 5 years of age; and about 7.2 deaths per 100,000 people per year in adults over 65 years of age. In addition, RSV infections cannot produce permanent immunity and therefore cannot protect children from reinfection. RSV has become one of the most important causes of acute lower respiratory infections worldwide in children under 5 years of age and elderly people over 65 years of age with memory immunodeficiency, and has become a recognized global health problem.

The prevention of viral diseases in general is done by vaccines. However, since RSV has been isolated in 1957, there have been no approved vaccines that can be used for the prevention in the past decades. Previous vaccine research and development has resulted in cases where the vaccine not only failed to play a protective role, but exacerbated the pneumonia caused by RSV infections, leading to the deaths of children. It can be seen that the druggability risk in vaccine research and development is still very high. As a result, more and more attention is focused on the development of RSV antibody drugs. Currently, the only preventive drug against the disease is a monoclonal antibody Palivizumab, which was first approved by the FDA in 1998. Therefore, there is an urgent need to develop new anti-RSV drugs, especially those that can treat RSV infections.

RSV is a non-segmented single-stranded negative-strand RNA virus belonging to the family *Paramyxoviridae,* the genus *Pneumovirus.* The RSV particle contains an envelope. The RSV particle is composed of an envelope, a nucleocapsid, and a core. The RSV genome is 15.2 kb in full length and transcribes 10 genes (NS1, NS2, N, P, M, SH, G, F, M2 and L from the 3' end to the 5' end), which encode 11 proteins. Among these genes, M2 contains two open reading frames, which encode two proteins, M2-1 and M2-2; each of the remaining genes encodes one protein. NS1 and NS2 are non-structural proteins; N (nucleocapsid protein), P (phosphoprotein) and L (polymerase subunit protein) are nucleocapsid proteins; M, M2-1 and M2-2 are matrix proteins; F (fusion protein), G (adhesion protein) and SH (small hydrophobin) are transmembrane glycoproteins. The G protein and F protein on the surface of the RSV particles mediate the adsorption and fusion between the virus and host cells, and between virus-infected cells and uninfected cells. Moreover, they are the primary protective antigens of RSV, which can induce the body to produce protective neutralizing antibodies. The research results show that G protein is highly variable between the two subtypes, and induces the body to produce type-specific neutralizing antibodies, which do not have an extensive protective effect. F protein is a highly conserved protein, and the protective antibodies induced thereby are broad-spectrum neutralizing antibodies, which can inhibit both RSV A and B infections. F protein is a potential effective target protein for the research on RSV preventive and therapeutic drugs and vaccines. F protein is only active when it is cleaved by the host protease into two fragments, F1 (48 kDa) and F2 (26 kDa). F protein typically forms a trimeric structure of F1-F2 heterodimers to play a fusion role. On the F protein, there are currently 6 epitopes that are believed to be capable of producing neutralizing antibodies, i.e., epitopes Ø, I, II, III, IV and V.

**SUMMARY OF THE INVENTION**

In one aspect, the present invention provides an antibody or an antigen-binding fragment thereof that specifically binds to RSV, comprising:

(1) a CDR-H1 set forth in SEQ ID NO: 1, a CDR-H2 set forth in SEQ ID NO: 2, a CDR-H3 set forth in SEQ ID NO: 3, a CDR-L1 set forth in SEQ ID NO: 4, a CDR-L2 set forth in SEQ ID NO: 5, and a CDR-L3 set forth in SEQ ID NO: 6;
(2) a CDR-H1 set forth in SEQ ID NO: 9, a CDR-H2 set forth in SEQ ID NO: 10, a CDR-H3 set forth in SEQ ID NO: 11, a CDR-L1 set forth in SEQ ID NO: 12, a CDR-L2 set forth in SEQ ID NO: 13, and a CDR-L3 set forth in SEQ ID NO: 14;
(3) a CDR-H1 set forth in SEQ ID NO: 17, a CDR-H2 set forth in SEQ ID NO: 18, a CDR-H3 set forth in SEQ ID NO: 19, a CDR-L1 set forth in SEQ ID NO: 20, a CDR-L2 set forth in SEQ ID NO: 21, and a CDR-L3 set forth in SEQ ID NO: 22; or
(4) a CDR-H1 set forth in SEQ ID NO: 25, a CDR-H2 set forth in SEQ ID NO: 26, a CDR-H3 set forth in SEQ ID NO: 27, a CDR-L1 set forth in SEQ ID NO: 28, a CDR-L2 set forth in SEQ ID NO: 29, and a CDR-L3 set forth in SEQ ID NO: 30.

In one embodiment, the antibody or the antigen-binding fragment thereof of the present invention comprises:

(1) a VH set forth in SEQ ID NO: 7 and a VL set forth in SEQ ID NO: 8;
(2) a VH set forth in SEQ ID NO: 15 and a VL set forth in SEQ ID NO: 16;
(3) a VH set forth in SEQ ID NO: 23 and a VL set forth in SEQ ID NO: 24; or
(4) a VH set forth in SEQ ID NO: 31 and a VL set forth in SEQ ID NO: 32.

In one embodiment, the antibody or the antigen-binding fragment thereof of the present invention comprises:

(1) a heavy chain constant region set forth in SEQ ID NO: 35, and
(2) a light chain constant region set forth in SEQ ID NO: 33 or 34.

In one embodiment, the antibody or the antigen-binding fragment thereof of the present invention comprises an $\alpha$ heavy chain, a $\delta$ heavy chain, an $\varepsilon$ heavy chain, a $\gamma$ heavy chain or a $\mu$ heavy chain. In one embodiment, the antibody or the antigen-binding fragment thereof of the present invention belongs to an IgG1, IgG2, IgG3 or IgG4 subclass. In one embodiment, the antibody or the antigen-binding fragment thereof of the present invention comprises a $\lambda$ light chain or a $\kappa$ light chain. In one embodiment, the antibody or the antigen-binding fragment thereof of the present invention is a full-length antibody. In one embodiment, the antibody or the antigen-binding fragment thereof of the present invention is an antibody fragment selected from Fv, scFv, Fab, Fab', F(ab')$_2$ and xFab. In one embodiment, the antibody or the antigen-binding fragment thereof of the present invention is a chimeric antibody or a human antibody or an antigen-binding fragment thereof.

In one embodiment, the antibody or the antigen-binding fragment thereof of the present invention has one or more of properties of:

(1) being capable of specifically binding to an RSV A2 pre-F protein;
(2) being capable of specifically binding to an RSV A2 pre-F protein with an EC50 value less than 60, 55, 50, 45, 40, 35, 34, 33, 32, 31, 30, 29, 28, 27, 26, 25, 24, 23, 22, 21, 20, 19, 18, 17, 16, 15, 14, 13, 12, 11 or 10 ng/mL, or with an EC50 value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that of MEDI8897;
(3) being capable of specifically binding to an RSV A2 pre-F protein with a KD value less than 5.0, 1.0, 0.9, 0.8, 0.7, 0.6, 0.5, 0.4, 0.3, 0.2, 0.1, 0.09, 0.08, 0.07, 0.06, 0.05, 0.04, 0.03, 0.02, 0.01, 0.009, 0.008, 0.007, 0.006, 0.005, 0.004, 0.003, 0.002 or 0.001 nM, or with a KD value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that of MEDI8897;
(4) being capable of inhibiting RSV (e.g., subgroup A and/or subgroup B) from infecting a host cell;
(5) being capable of inhibiting an RSV subgroup A strain (e.g., A2) from infecting a host cell with an IC50 value less than 100, 90, 80, 70, 60, 50, 45, 40, 35, 30, 25, 20, 15, 10, 9, 8, 7, 6, 5, 4, 3, 2, 1 or 0.5 ng/mL, or with an IC50 value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that MEDI8897;
(6) being capable of inhibiting an RSV subgroup B strain (e.g., B9320) from infecting a host cell with an IC50 value less than 160, 150, 140, 130, 120, 110, 100, 90, 80, 70, 60, 50, 40, 30, 20, 15, 10, 9, 8, 7, 6, 5, 4 or 3 ng/mL, or with an IC50 value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that of MEDI8897;
(7) being capable of inhibiting an RSV subgroup B strain (e.g., B18537) from infecting a host cell with an IC50 value less than 50, 45, 40, 35, 30, 20, 15, 10, 9, 8, 7, 6, 5, 4, 3, 2 or 1 ng/mL, or with an IC50 value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that of MEDI8897;
(8) being capable of competing for or inhibiting the binding of MEDI8897 to an RSV A2 pre-F protein;
(9) being capable of inhibiting the binding of MEDI8897 to an RSV A2 pre-F protein by 1% to 100%, e.g., at least 5%, 10%, 15%, 20%, 25%, 30%, 35%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100%;
(10) being capable of competing for or inhibiting the binding of MK-1654 to an RSV A2 pre-F protein;
(11) being capable of inhibiting the binding of MK-1654 to an RSV A2 pre-F protein by 1% to 100%, e.g., at least 5%, 10%, 15%, 20%, 25%, 30%, 35%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100%;

(12) being capable of competing for or inhibiting the binding of Motavizumab to an RSV A2 pre-F protein;

(13) being capable of inhibiting the binding of Motavizumab to an RSV A2 pre-F protein by 1% to 100%, e.g., at least 5%, 10%, 15%, 20%, 25%, 30%, 35%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100%;

(14) being capable of competing for or inhibiting the binding of MPE8 to an RSV A2 pre-F protein;

(15) being capable of inhibiting the binding of MPE8 to an RSV A2 pre-F protein by 1% to 100%, e.g., at least 5%, 10%, 15%, 20%, 25%, 30%, 35%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100%;

(16) being capable of binding to an epitope Ø of an RSV A2 pre-F protein;

(17) being capable of binding to an epitope IV of an RSV A2 pre-F protein;

(18) being capable of binding to epitopes Ø and IV of an RSV A2 pre-F protein;

(19) being capable of binding to an epitope II of an RSV A2 pre-F protein;

(20) being capable of binding to an epitope III of an RSV A2 pre-F protein; and/or

(21) being capable of binding to epitopes II and III of an RSV A2 pre-F protein.

In one aspect, the present invention provides a polynucleotide encoding the antibody or the antigen-binding fragment thereof of the present invention.

In one embodiment, the polynucleotide of the present invention comprises:

(1) SEQ ID NOs: 36 and 37;
(2) SEQ ID NOs: 38 and 39;
(3) SEQ ID NOs: 40 and 41; or
(4) SEQ ID NOs: 42 and 43.

In one aspect, the present invention provides a vector comprising the polynucleotide of the present invention.

In one aspect, the present invention provides a host cell comprising the polynucleotide or the vector of the present invention. In one embodiment, the host cell is a eukaryotic cell. In one embodiment, the host cell is a CHO cell.

In one aspect, the present invention provides a method for generating an antibody or an antigen-binding fragment thereof, which method comprises:

(a) culturing the host cell of the present invention under conditions suitable for expression of the antibody or the antigen-binding fragment thereof, and

(b) optionally, recovering the antibody or the antigen-binding fragment thereof.

In one aspect, the present invention provides a composition comprising the antibody or the antigen-binding fragment thereof of the present invention.

In one aspect, the present invention provides the antibody or the antigen-binding fragment thereof or the composition of the present invention, for use as a medicament.

In one aspect, the present invention provides the antibody or the antigen-binding fragment thereof or the composition of the present invention, for use in the treatment of a disease. In one embodiment, the disease is a lower respiratory tract infection. In one embodiment, the disease is a disease caused by an RSV infection.

In one aspect, the present invention provides use of the antibody or the antigen-binding fragment thereof or the composition of the present invention for the manufacture of a medicament for treating a disease. In one embodiment, the disease is a lower respiratory tract infection.

In one aspect, the present invention provides a method for treating a disease in an individual, which method comprises administering to the individual a therapeutically effective amount of the antibody or the antigen-binding fragment thereof or the composition of the present invention. In one embodiment, the disease is a lower respiratory tract infection.

## BRIEF DESCRIPTION OF THE DRAWINGS

FIG. 1-1 and FIG. 1-2 show antigen-binding curves of antibodies of the present invention (ELISA).
FIG. 2-1 and FIG. 2-2 show antigen-binding curves of antibodies of the present invention (BA).
FIG. 3-1 and FIG. 3-2 show virus neutralization curves of antibodies of the present invention.

FIG. 4 shows a schematic diagram of competitive binding epitope assay of antibodies of the present invention.

**DETAILED DESCRIPTION OF EMBODIMENTS**

For purposes herein, the "acceptor human framework" refers to a framework comprising an amino acid sequence of a light chain variable domain (VL) framework or a heavy chain variable domain (VH) derived from a human immunoglobulin framework or a human consensus framework as defined below. An acceptor human framework "derived" from a human immunoglobulin framework or a human consensus framework may comprise the same amino acid sequence thereto, or it may contain amino acid sequence variations. In some embodiments, the number of amino acid variations is 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or the human consensus framework sequence.

The "affinity" refers to the strength of the sum total of non-covalent interactions between a single binding site of a molecule (e.g., an antibody) and its binding partner (e.g., an antigen). Unless otherwise indicated, as used herein, the "binding affinity" refers to an intrinsic binding affinity that reflects a 1 : 1 interaction between members of a binding pair (e.g., antibody and antigen). The affinity of a molecule X for its partner Y can generally be expressed by the dissociation constant (Kd). Affinity can be measured by common methods known in the art, including those described herein. Specific illustrative and exemplary embodiments for measuring binding affinity are described below.

An "affinity-matured" antibody refers to an antibody that has one or more alterations in one or more hypervariable regions (HVRs), compared to a parent antibody that does not possess such alterations, such alterations resulting in an improvement in the affinity of the antibody for an antigen.

The terms "anti-RSV antibody" and "antibody that binds to RSV" refer to an antibody that is capable of binding to RSV with sufficient affinity such that the antibody can be used as a diagnostic, preventive, and/or therapeutic agent in targeting RSV. In one embodiment, the extent of binding of the anti-RSV antibody to an unrelated, non-RSV protein is less than about 10% of the binding of the antibody to RSV, as measured, for example, by radioimmunoassay (RIA). In certain embodiments, the antibody that binds to RSV has a dissociation constant (Kd) of $\leq 1\ \mu M$, $\leq 100\ nM$, $\leq 10\ nM$, $\leq 1\ nM$, $\leq 0.1\ nM$, $\leq 0.01\ nM$, or $\leq 0.001\ nM$ (e.g. $10^{-8}$ M or less, e.g., $10^{-8}$ M to $10^{-13}$ M, e.g., $10^{-9}$ M to $10^{-13}$ M). In certain embodiments, the "anti-RSV antibody" and the "antibody that binds to RSV" are "anti-RSV pre-F antibody" and "antibody that binds to RSV pre-F", particularly "anti-RSV A2 pre-F antibody" and "antibody that binds to RSV A2 pre-F". In certain embodiments, the anti-RSV antibody binds to a pre-F epitope that is conserved in different RSV strains. In a preferred embodiment, the "anti-RSV antibody", the "antibody that specifically binds to RSV" and the "antibody that binds to RSV" refer to an antibody that specifically binds to RSV or an antigen thereof or an epitope thereof with a binding affinity of a $K_D$ value of $1.0 \times 10^{-8}$ mol/l or less, in one embodiment, a $K_D$ value of $1.0 \times 10^{-9}$ mol/l or less, in one embodiment, a $K_D$ value of $1.0 \times 10^{-9}$ mol/l to $1.0 \times 10^{-13}$ mol/l. In this context, the binding affinity is determined using standard binding assays, such as surface plasmon resonance (BIAcore®, GE-Healthcare Uppsala, Sweden; SARTORIUS, Octet® R8), for example, using an RSV A2 pre-F protein.

The term "antibody" is used herein in the broadest sense and encompasses various antibody structures including, but not limited to, monoclonal antibodies, polyclonal antibodies, multispecific antibodies (such as bispecific antibodies) and antibody fragments, so long as they exhibit the desired antigen-binding activity.

The "antibody fragment" refers to a molecule that is different from an intact antibody and comprises a portion of an intact antibody that binds to an antigen to which the intact antibody binds. Examples of antibody fragments include, but are not limited to, Fv, Fab, Fab', Fab'-SH, F(ab')$_2$, xFab; diabodies; linear antibodies; single-chain antibody molecules (e.g. scFv); and multispecific antibodies formed from antibody fragments.

The term "epitope" refers to a site on an antigen (either proteinaceous or non-proteinaceous) to which an anti-RSV antibody binds. Epitopes can be formed from a string of contiguous amino acids (linear epitopes) or comprise non-contiguous amino acids (conformational epitopes), for example, brought into spatial proximity by folding of an antigen (i.e., by tertiary folding of a proteinaceous antigen). Linear epitopes are typically still bound by anti-RSV antibodies after exposure of proteinaceous antigens to denaturants, whereas conformational epitopes are typically destroyed after treatment with denaturants. An epitope comprises at least 3, at least 4, at least 5, at least 6, at least 7, or 8-10 amino acids in a unique spatial conformation.

Screening for antibodies that bind to a particular epitope (i.e., those that bind to the same epitope) can be performed using routine methods in the art, such as, for example, but not limited to, alanine scanning, peptide imprinting (see Meth. Mol. Biol., 248 (2004) 443-463), peptide cleavage analysis, epitope excision, epitope extraction, chemical modification of antigens (see Prot. Sci., 9 (2000) 487-496), and cross-blocking (see "Antibodies", Harlow and Lane (Cold Spring Harbor Press, Cold Spring Harb., NY)).

Antigen structure-based antibody profiling (ASAP) (also referred to as modification-assisted profiling (MAP)) allows a population of monoclonal antibodies that specifically bind to RSV to be binned on the basis of the binding profile of each antibody from the population to chemically or enzymatically modified antigen surfaces (see, e.g., US 2004/0101920).

The antibodies in each bin bind to the same epitope, which can be a unique epitope that is distinct from or partially overlaps with the epitope represented by the other bin.

Competitive binding can also be used to readily identify whether an antibody binds to or competes for binding to the same RSV epitope as a reference anti-RSV antibody. For example, an antibody that "binds to the same epitope" as a reference anti-RSV antibody refers to an antibody that blocks binding of the reference anti-RSV antibody to its antigen in a competition assay by 50% or more, and conversely, the reference antibody blocks binding of the antibody to its antigen in a competition assay by 50% or more. For another example, to determine whether the antibody binds to the same epitope as the reference anti-RSV antibody, the reference antibody is allowed to bind to RSV under saturation conditions. After removal of the excess reference anti-RSV antibody, the ability of the anti-RSV antibody in question to bind to RSV is evaluated. If an anti-RSV antibody is capable of binding to RSV after saturation binding of a reference anti-RSV antibody, it can be concluded that the anti-RSV antibody in question binds to a different epitope than the reference anti-RSV antibody. However, if the anti-RSV antibody in question fails to bind to RSV after saturation binding of the reference anti-RSV antibody, the anti-RSV antibody in question may bind to the same epitope as that bound by the reference anti-RSV antibody. To confirm whether the antibody in question binds to the same epitope or is simply hindered by steric reasons, routine experiments (e.g., peptide mutagenesis and binding analysis using ELISA, RIA, surface plasmon resonance, flow cytometry or any other quantitative or qualitative antibody binding assays available in the art). This assay should be performed in two settings, that is, both antibodies are used as saturating antibodies. If only the first (saturating) antibody is capable of binding to RSV in both settings, it can be concluded that the anti-RSV antibody in question competes for the binding to RSV with the reference anti-RSV antibody.

In some embodiments, two antibodies are considered to bind to the same or overlapping epitope if a 1, 5, 10, 20 or 100-fold excess of one antibody inhibits the binding of the other by at least 50%, at least 75%, at least 90%, or even 99% or more, as measured in a competitive binding assay (see, e.g., Junghans et al., Cancer Res. 50 (1990) 1495-1502).

In some embodiments, two antibodies are considered to bind to the same epitope if an amino acid mutation in an antigen that reduces or eliminates the binding of one antibody also substantially reduces or eliminates the binding of the other. Two antibodies are considered to have "overlapping epitopes" if only a subset of amino acid mutations that reduce or eliminate the binding of one antibody reduce or eliminate the binding of the other.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are 5 major classes of antibodies: IgA, IgD, IgE, IgG and IgM, and several of these can be further divided into subclasses (isotypes), e.g., IgG1, $IgG_2$, $IgG_3$, $IgG_4$, $IgA_1$, and $IgA_2$. In certain embodiments, the antibody is of the IgG4 isotype with an S228P mutation in the hinge region to improve the stability of the IgG4 antibody. The heavy chain constant domains that correspond to different classes of immunoglobulins are referred to as $\alpha$, $\delta$, $\varepsilon$, $\gamma$, and $\mu$, respectively.

As used herein, the term "cytotoxic agent" refers to a substance that inhibits or prevents cellular functions and/or causes cell death or destruction. Cytotoxic agents include, but are not limited to: radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu), chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalators); growth inhibitors; enzymes and fragments thereof, such as nucleolytic enzyme; antibiotics; toxins, such as small molecule toxins or enzymatically active toxins of bacterial, fungal, plant or animal origin, including fragments and/or variants thereof; and various anti-tumor or anti-cancer agents disclosed below.

The "effector function" refers to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down-regulation of cell surface receptors (e.g., B-cell receptors); and B-cell activation.

An "effective amount" of an agent (e.g., a pharmaceutical formulation) refers to an amount effective, at dosages and for periods of time necessary, to achieve the desired therapeutic or preventive result.

The term "Fc region" herein is used to define a C-terminal region of an immunoglobulin heavy chain that contains at least a portion of the constant region. The term includes natural sequence Fc regions and variant Fc regions. In one embodiment, a human IgG heavy chain Fc region extends from Cys226, or from Pro230, to the carboxyl terminus of the heavy chain. However, the C-terminal lysine (Lys447) of the Fc region may or may not be present. In one embodiment, the anti-RSV antibody as described herein is of the IgG1 isotype and comprises a constant heavy chain domain of SEQ ID NO: 35. In one embodiment, it additionally comprises a C-terminal lysine (Lys447). In one embodiment, the anti-RSV antibody as described herein is of the IgG4 isotype. In one embodiment, it additionally comprises a C-terminal lysine (Lys447). Unless otherwise specified herein, numbering of amino acid residues in the Fc region or constant region is according to the EU numbering system, also referred to as the EU index, as recorded in Kabat et al., Sequences of Proteins of Immunological Interest, 5th edition, Public Health Service, National Institutes of Health, Bethesda, MD, 1991.

The "framework" or "FR" refers to variable domain residues other than hypervariable region (HVR) residues. The FR of a variable domain generally consists of four FR domains: FR1, FR2, FR3 and FR4. Thus, the HVR and FR sequences generally appear in the following sequence in VH (or VL): FR1-H1(L1)-FR2-H2(L2)-FR3-H3(L3)-FR4.

The terms "full-length antibody", "intact antibody" and "whole antibody" are used interchangeably herein and refer to an antibody having a structure substantially similar to that of a natural antibody or having a heavy chain containing an Fc region as defined herein.

The terms "host cell", "host cell line" and "host cell culture" are used interchangeably and refer to a cell into which an exogenous nucleic acid has been introduced, including the progeny of such cells. Host cells include "transformants" and "transformed cells", which include the primary transformed cells and progeny derived therefrom, regardless of the number of passages. The progeny may not be completely identical in nucleic acid content to a parent cell, but may contain mutations. The mutant progeny having the same function or biological activity as those screened or selected for in the initially transformed cells are included herein.

The "human antibody" refers to an antibody possessing an amino acid sequence corresponding to the amino acid sequence of an antibody generated by a human or a human cell or derived from a non-human source using a human antibody repertoire or other human antibody coding sequences. This definition of a human antibody explicitly excludes humanized antibodies comprising non-human antigen-binding residues. In certain embodiments, the human antibody is derived from a non-human transgenic mammal, e.g., a mouse, a rat, or a rabbit. In certain embodiments, the human antibody is derived from a hybridoma cell line.

The "human consensus framework" refers to a framework that represents the most frequently occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, 5th edition, NIH Publication 91-3242, Bethesda MD (1991), Vols. 1-3. In one embodiment, for VL, the subgroup is subgroup κI, as recorded in Kabat et al., supra. In one embodiment, for VH, the subgroup is subgroup III, as recorded in Kabat et al., supra.

A "humanized" antibody refers to a chimeric antibody comprising amino acid residues from non-human HVRs and amino acid residues from human FRs. In certain embodiments, the humanized antibody may comprise at least one, and usually two, substantially entire variable domains, in which all or substantially all of the HVRs (e.g., CDRs) correspond to those of a non-human antibody, and all or substantially all of the FRs correspond to those of a human antibody. Optionally, a humanized antibody may comprise at least a portion of an antibody constant region derived from a human antibody. A "humanized form" of an antibody (e.g., a non-human antibody) refers to an antibody that has undergone humanization.

As used herein, the term "hypervariable region" or "HVR" refers to each of the regions of an antibody variable domain which is hypervariable in sequence ("complementarity determining region" or "CDR") and/or forms a structurally defined loop ("hypervariable loop") and/or contains an antigen contact residue ("antigen contact"). In general, an antibody comprises six HVRs: three in VH (H1, H2, H3) and three in VL (L1, L2, L3). Exemplary HVRs herein include:

(a) hypervariable loops occurring at amino acid residues 26-32 (L1), 50-52 (L2), 91-96 (L3) 26-32 (H1), 53-55 (H2) and 96-101 (H3) (Chothia and Lesk, J. Mol. Biol., 196: 901-917 (1987));
(b) CDRs occurring at amino acid residues 24-34 (L1), 50-56 (L2), 89-97 (L3) 31-35b (H1), 50-65 (H2) and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3) 30-35b (H1), 47-58 (H2) and 93-101 (H3) (MacCallum et al., J. Mol. Biol., 262: 732-745 (1996)); and
(d) combinations of (a), (b) and/or (c), including HVR amino acid residues 46-56 (L2), 47-56 (L2), 48-56 (L2), 49-56 (L2), 26-35 (H1) 26-35b (H1), 49-65 (H2), 93-102 (H3) and 94-102 (H3).

In one embodiment, the HVR residues comprise those identified in the description of the amino acid sequence below.

Unless otherwise indicated, HVR residues and other residues in the variable domain (e.g., FR residues) are numbered herein according to Kabat et al., supra.

The "immunoconjugate" refers to an antibody conjugated to one or more heterologous molecules, including, but not limited to, a cytotoxic agent.

The "individual" or "subject" refers to a mammal. Mammals include, but are not limited to, domesticated animals (e.g., cows, sheep, cats, dogs, and horses), primates (e.g., human and non-human primates such as monkeys), rabbits, and rodents (e.g., mice and rats). In certain embodiments, the individual or subject is a human.

An "isolated" antibody is an antibody that has been separated from components of its natural environment. In some embodiments, the antibody is purified to greater than 95% or 99% purity, as determined by, for example, electrophoresis (e.g., SDS-PAGE, isoelectric focusing (IEF), capillary electrophoresis) or chromatography (e.g., ion exchange or reversed-phase HPLC methods). For a review of methods for evaluating antibody purity, see, e.g., Flatman et al., J. Chromatogr., B 848: 79-87 (2007).

An "isolated" nucleic acid refers to a nucleic acid molecule that has been separated from components of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The "isolated nucleic acid encoding an anti-RSV antibody" refers to one or more nucleic acid molecules encoding antibody heavy and light chains (or fragments thereof), including such nucleic acid molecules in a single vector or in different vectors, and such nucleic acid molecules present at one or more locations in a host cell.

As used herein, the term "monoclonal antibody" refers to an antibody obtained from a population of substantially homogeneous antibodies, i.e., individual antibodies comprising the population are identical and/or bind to the same epitope, except for possible variant antibodies, e.g., containing naturally occurring mutations or arising during generation of a monoclonal antibody preparation, such variants generally being present in minor amounts. In contrast to polyclonal antibody preparations that typically include different antibodies directed against different determinants (epitopes), each monoclonal antibody of a monoclonal antibody preparation is directed against a single determinant on an antigen. As such, the modifier "monoclonal" indicates the character of the antibody obtained from a population of substantially homogeneous antibodies, and is not to be construed as requiring the generation of the antibody by any particular method. For example, monoclonal antibodies to be used in accordance with the present invention may be generated by a variety of techniques including, but not limited to, hybridoma methods, recombinant DNA methods, phage display methods, and methods of using transgenic animals containing all or part of human immunoglobulin loci, and such methods and other exemplary methods for generating monoclonal antibodies are described herein.

The "naked antibody" refers to an antibody that is not conjugated to a heterologous moiety (e.g., cytotoxic moiety) or a radioactive label. Naked antibodies can be present in pharmaceutical formulations.

The "natural antibody" refers to naturally occurring immunoglobulin molecules with different structures. For example, a natural IgG antibody is a heterotetrameric glycoprotein of about 150,000 daltons, consisting of two identical light chains and two identical heavy chains that are disulfide-bonded. From the N-terminus to the C-terminus, each heavy chain has a variable region (VH), also referred to as a variable heavy domain or a heavy chain variable domain, followed by three constant domains (CH1, CH2 and CH3). Similarly, from the N-terminus to the C-terminus, each light chain has a variable region (VL), also referred to as a variable light domain or a light chain variable domain, followed by a constant light (CL) domain. Antibody light chains can be assigned to one of two types, referred to as kappa ($\kappa$) and lambda ($\lambda$), based on their constant domain amino acid sequences.

The term "package insert" is used to refer to the instructions for use typically included in commercial packages of therapeutic products that contain information regarding indications, usage, dosage, administration, combination therapies, contraindications and/or warnings involving the use of such therapeutic products.

The "Percent (%) amino acid sequence identity" with respect to a reference polypeptide sequence is defined as the percentage of amino acid residues in a candidate sequence that are identical to amino acid residues in the reference polypeptide sequence, after the sequences are aligned and gaps are introduced, if necessary, to achieve the maximum percent sequence identity. Alignment for purposes of determining percent amino acid sequence identity can be performed in a variety of ways within the skill in the art, for example, using publicly available computer software such as BLAST, BLAST-2, Clustal W, Megalign (DNASTAR) software or FASTA package. A person skilled in the art can determine appropriate parameters for aligning sequences, including any algorithm needed to achieve the maximal alignment over the full length of the sequences being compared. However, for purposes herein, % amino acid sequence identity values are generated using the ggsearch program of the FASTA package of version 36.3.8c or later and the BLOSUM50 comparison matrix. The FASTA package is recorded by W. R. Pearson and D. J. Lipman (1988) "Improved Tools for Biological Sequence Analysis", PNAS 85: 2444-2448; W. R. Pearson (1996) "Effective protein sequence comparison" Meth. Enzymol. 266: 227-258; and Pearson et al. (1997) Genomics 46: 24-36 and is publicly available from http://fasta. bioch.virginia.edu/fasta_www2/fasta_down.shtml. Alternatively, a public server accessible at http://fasta.bioch.virginia. edu/fasta_www2/index.cgi can be used to compare sequences, and a ggsearch (global protein:protein) program with default options (BLOSUM50; gap open: -10; gap extension: -2; Ktup = 2) is used to ensure that a global alignment rather than a local alignment is performed. The percent amino acid identity is given in the output alignment header.

The term "pharmaceutical formulation" refers to a preparation which is in such form as to permit the biological activity of an active ingredient contained therein to be effective and which contains no additional components that are unacceptably toxic to a subject to which the formulation would be administered.

The "pharmaceutically acceptable carrier" refers to an ingredient in a pharmaceutical formulation, other than an active ingredient, which is nontoxic to a subject. Pharmaceutically acceptable carriers include, but are not limited to, buffers, excipients, stabilizers, or preservatives.

As used herein, "treatment" (and grammatical variations thereof) refers to clinical intervention that attempts to alter the natural course of the individual being treated, and may be performed either for prevention or during the course of clinical pathology. Desired effects of treatment include, but are not limited to, prevention of occurrence or recurrence of diseases, alleviation of symptoms, diminishment/reduction of any direct or indirect pathological consequences of diseases, prevention of metastasis, decrease in the rate of disease progression, amelioration or palliation of the disease state, and remission or improved prognosis. In some embodiments, the antibody of present the invention is used to delay the development of a disease or to slow the progression of a disease.

The term "prevention" as used herein includes slowing the onset of a disease, reducing the risk of developing a disease, inhibiting or delaying the manifestation or development of symptoms associated with a disease, reducing the severity of subsequent contraction or the development of a disease, ameliorating related symptoms, and inducing immunity to prevent a disease.

The term "variable region" or "variable domain" refers to a domain of an antibody heavy or light chain that is involved in the binding of the antibody to an antigen. The heavy and light chain variable domains (VH and VL, respectively) of a natural antibody generally have similar structures, with each domain containing four conserved framework regions (FRs) and three hypervariable regions (HVRs) (see, e.g., Kindt et al., Kuby Immunology, 6th edition, W.H. Freeman and Co., page 91 (2007)). A single VH or VL domain may be sufficient to confer antigen-binding specificity. In addition, antibodies that bind to a particular antigen may be isolated using a VH or VL domain from an antibody that binds to the antigen to screen a library of complementary VL or VH domains, respectively. See, e.g., Portolano et al., J. Immunol. 150: 880-887 (1993); Clarkson et al., Nature 352: 624-628 (1991).

As used herein, the term "vector" refers to a nucleic acid molecule capable of propagating another nucleic acid to which it is linked. This term includes a vector as a self-replicating nucleic acid structure and a vector incorporated into the genome of a host cell into which it has been introduced. Certain vectors are capable of directing the expression of nucleic acids to which they are operably linked. Such vectors are referred to herein as "expression vectors".

## I. Compositions and methods

In one aspect, the present invention provides an isolated antibody that binds to RSV.

In certain embodiments, an antibody that binds to RSV is provided. The antibody of the present invention is useful, for example, for the diagnosis or treatment of RSV infections, such as a lower respiratory tract infection.

## A. Exemplary anti-RSV antibodies

In certain embodiments, an anti-RSV antibody is provided, wherein the antibody:

i) binds to RSV, particularly an RSV pre-F protein, more particularly an RSV A2 pre-F protein; and/or
ii) inhibits RSV, particularly RSV A1, RSV B9320 and/or RSV B18537, from infecting a host cell, such as a Hep2 cell.

In one aspect, the present invention provides an anti-RSV antibody, which comprises at least one, two, three, four, five or six CDRs selected from: (a) a CDR-H1 comprising an amino acid sequence of SEQ ID NO: 1; (b) a CDR-H2 comprising an amino acid sequence of SEQ ID NO: 2; (c) a CDR-H3 comprising an amino acid sequence of SEQ ID NO: 3; (d) a CDR-L1 comprising an amino acid sequence of SEQ ID NO: 4; (e) a CDR-L2 comprising an amino acid sequence of SEQ ID NO: 5; and (f) a CDR-L3 comprising an amino acid sequence of SEQ ID NO: 6.

In one aspect, the present invention provides an antibody, which comprises at least one, at least two or all three VH CDR sequences selected from: (a) a CDR-H1 comprising an amino acid sequence of SEQ ID NO: 1; (b) a CDR-H2 comprising an amino acid sequence of SEQ ID NO: 2; and (c) a CDR-H3 comprising an amino acid sequence of SEQ ID NO: 3. In one embodiment, the antibody comprises a CDR-H3 comprising an amino acid sequence of SEQ ID NO: 3. In another embodiment, the antibody comprises a CDR-H3 comprising an amino acid sequence of SEQ ID NO: 3 and a CDR-L3 comprising an amino acid sequence of SEQ ID NO: 6. In yet another embodiment, the antibody comprises a CDR-H3 comprising an amino acid sequence of SEQ ID NO: 3, a CDR-L3 comprising an amino acid sequence of SEQ ID NO: 6, and a CDR-H2 comprising an amino acid sequence of SEQ ID NO: 2. In yet another embodiment, the antibody comprises (a) a CDR-H1 comprising an amino acid sequence of SEQ ID NO: 1; (b) a CDR-H2 comprising an amino acid sequence of SEQ ID NO: 2; and (c) a CDR-H3 comprising an amino acid sequence of SEQ ID NO: 3.

In another aspect, the present invention provides an antibody, which comprises at least one, at least two or all three VL CDR sequences selected from: (a) a CDR-L1 comprising an amino acid sequence of SEQ ID NO: 4; (b) a CDR-L2 comprising an amino acid sequence of SEQ ID NO: 5; and (c) a CDR-L3 comprising an amino acid sequence of SEQ ID NO: 6. In one embodiment, the antibody comprises (a) a CDR-L1 comprising an amino acid sequence of SEQ ID NO: 4; (b) a CDR-L2 comprising an amino acid sequence of SEQ ID NO: 5; and (c) a CDR-L3 comprising an amino acid sequence of SEQ ID NO: 6.

In another aspect, the antibody of the present invention comprises (a) a VH domain comprising at least one, at least two or all three VH CDR sequences selected from: (i) a CDR-H1 comprising an amino acid sequence of SEQ ID NO: 1, (ii) a CDR-H2 comprising an amino acid sequence of SEQ ID NO: 2, and (iii) a CDR-H3 comprising an amino acid sequence of SEQ ID NO: 3; and (b) a VL domain comprising at least one, at least two, or all three VL CDR sequences selected from: (i) a CDR-L1 comprising an amino acid sequence of SEQ ID NO: 4, (ii) a CDR-L2 comprising an amino acid sequence of SEQ ID NO: 5, and (c) a CDR-L3 comprising an amino acid sequence of SEQ ID NO: 6.

In another aspect, the present invention provides an antibody, which comprises (a) a CDR-H1 comprising an amino acid sequence of SEQ ID NO: 1; (b) a CDR-H2 comprising an amino acid sequence of SEQ ID NO: 2; (c) a CDR-H3 comprising an amino acid sequence of SEQ ID NO: 3; (d) a CDR-L1 comprising an amino acid sequence of SEQ ID NO: 4; (e) a CDR-L2 comprising an amino acid sequence of SEQ ID NO: 5; and (f) a CDR-L3 comprising an amino acid sequence selected from SEQ ID NO: 6.

In any of the above embodiments, the anti-RSV antibody is human or humanized. In one embodiment, the anti-RSV antibody comprises CDRs as in any of the above embodiments, and further comprises an acceptor human framework, such as a human immunoglobulin framework or a human consensus framework.

In another aspect, the anti-RSV antibody comprises a heavy chain variable domain (VH) sequence having at least 90%, 91%, 92%, 93%, 94%, 95%, 96%, 97%, 98%, 99% or 100% sequence identity to an amino acid sequence of SEQ ID NO: 7. In certain embodiments, the VH sequence having at least 90%, 91%, 92% 93%, 94%, 95%, 96%, 97%, 98% or 99% identity contains substitutions (e.g., conservative substitutions), insertions or deletions relative to a reference sequence, but the anti-RSV antibody comprising the sequence retains the ability to bind to RSV. In certain embodiments, a total of 1 to 10 amino acids are substituted, inserted and/or deleted in SEQ ID NO: 7. In certain embodiments, the substitutions, insertions or deletions occur in regions other than CDRs (i.e., in FRs). Optionally, the anti-RSV antibody comprises a VH sequence in SEQ ID NO: 7, including a post-translational modification of the sequence. In a particular embodiment, the VH comprises one, two or three CDRs selected from: (a) a CDR-H1 comprising an amino acid sequence of SEQ ID NO: 1, (b) a CDR-H2 comprising an amino acid sequence of SEQ ID NO: 2, and (c) a CDR-H3 comprising an amino acid sequence of SEQ ID NO: 3.

In another aspect, an anti-RSV antibody is provided, wherein the antibody comprises a light chain variable domain (VL) having at least 90%, 91%, 92%, 93%, 94%, 95%, 96%, 97%, 98%, 99% or 100% sequence identity to an amino acid sequence of SEQ ID NO: 8. In certain embodiments, the VL sequence having at least 90%, 91%, 92% 93%, 94%, 95%, 96%, 97%, 98% or 99% identity contains substitutions (e.g., conservative substitutions), insertions or deletions relative to a reference sequence, but the anti-RSV antibody comprising the sequence retains the ability to bind to RSV. In certain embodiments, a total of 1 to 10 amino acids are substituted, inserted and/or deleted in SEQ ID NO: 8. In certain embodiments, the substitutions, insertions or deletions occur in regions other than CDRs (i.e., in FRs). Optionally, the anti-RSV antibody comprises a VL sequence in SEQ ID NO: 8, including a post-translational modification of the sequence. In a particular embodiment, the VL comprises one, two or three CDRs selected from: (a) a CDR-L1 comprising an amino acid sequence of SEQ ID NO: 4; (b) a CDR-L2 comprising an amino acid sequence of SEQ ID NO: 5; and (c) a CDR-L3 comprising an amino acid sequence of SEQ ID NO: 6.

In another aspect, an anti-RSV antibody is provided, wherein the antibody comprises a VH as in any of the embodiments provided above, and a VL as in any of the embodiments provided above. In one embodiment, the antibody comprises a VH sequence in SEQ ID NO: 7 and a VL sequence in SEQ ID NO: 8, respectively, including post-translational modifications of those sequences.

In yet another aspect, the present invention provides an antibody that binds to the same epitope as the anti-RSV antibody provided herein. For example, in certain embodiments, an antibody that binds to the same epitope as an anti-RSV antibody comprising a VH sequence of SEQ ID NO: 7 and a VL sequence of SEQ ID NO: 8 is provided.

In one aspect, the present invention provides an anti-RSV antibody, which comprises at least one, two, three, four, five or six CDRs selected from: (a) a CDR-H1 comprising an amino acid sequence of SEQ ID NO: 9; (b) a CDR-H2 comprising an amino acid sequence of SEQ ID NO: 10; (c) a CDR-H3 comprising an amino acid sequence of SEQ ID NO: 11; (d) a CDR-L1 comprising an amino acid sequence of SEQ ID NO: 12; (e) a CDR-L2 comprising an amino acid sequence of SEQ ID NO: 13; and (f) a CDR-L3 comprising an amino acid sequence of SEQ ID NO: 14.

In one aspect, the present invention provides an antibody, which comprises at least one, at least two or all three VH CDR sequences selected from: (a) a CDR-H1 comprising an amino acid sequence of SEQ ID NO: 9; (b) a CDR-H2 comprising an amino acid sequence of SEQ ID NO: 10; and (c) a CDR-H3 comprising an amino acid sequence of SEQ ID NO: 11. In one embodiment, the antibody comprises a CDR-H3 comprising an amino acid sequence of SEQ ID NO: 11. In another embodiment, the antibody comprises a CDR-H3 comprising an amino acid sequence of SEQ ID NO: 11 and a CDR-L3 comprising an amino acid sequence of SEQ ID NO: 14. In yet another embodiment, the antibody comprises a CDR-H3 comprising an amino acid sequence of SEQ ID NO: 11, a CDR-L3 comprising an amino acid sequence of SEQ ID NO: 14, and a CDR-H2 comprising an amino acid sequence of SEQ ID NO: 10. In yet another embodiment, the antibody comprises (a) a CDR-H1 comprising an amino acid sequence of SEQ ID NO: 9; (b) a CDR-H2 comprising an amino acid sequence of SEQ ID NO: 10; and (c) a CDR-H3 comprising an amino acid sequence of SEQ ID NO: 11.

In another aspect, the present invention provides an antibody, which comprises at least one, at least two or all three VL CDR sequences selected from: (a) a CDR-L1 comprising an amino acid sequence of SEQ ID NO: 12; (b) a CDR-L2 comprising an amino acid sequence of SEQ ID NO: 13; and (c) a CDR-L3 comprising an amino acid sequence of SEQ ID NO: 14. In one embodiment, the antibody comprises (a) a CDR-L1 comprising an amino acid sequence of SEQ ID NO: 12; (b) a CDR-L2 comprising an amino acid sequence of SEQ ID NO: 13; and (c) a CDR-L3 comprising an amino acid sequence of SEQ ID NO: 14.

In another aspect, the antibody of the present invention comprises (a) a VH domain comprising at least one, at least two or all three VH CDR sequences selected from: (i) a CDR-H1 comprising an amino acid sequence of SEQ ID NO: 9, (ii) a CDR-H2 comprising an amino acid sequence of SEQ ID NO: 10, and (iii) a CDR-H3 comprising an amino acid sequence of SEQ ID NO: 11; and (b) a VL domain comprising at least one, at least two or all three VL CDR sequences selected from: (i) a CDR-L1 comprising an amino acid sequence of SEQ ID NO: 12, (ii) a CDR-L2 comprising an amino acid sequence of SEQ ID NO: 13, and (c) a CDR-L3 comprising an amino acid sequence of SEQ ID NO: 14.

In another aspect, the present invention provides an antibody, which comprises (a) a CDR-H1 comprising an amino acid sequence of SEQ ID NO: 9; (b) a CDR-H2 comprising an amino acid sequence of SEQ ID NO: 10; (c) a CDR-H3 comprising an amino acid sequence of SEQ ID NO: 11; (d) a CDR-L1 comprising an amino acid sequence of SEQ ID NO: 12; (e) a CDR-L2 comprising an amino acid sequence of SEQ ID NO: 13; and (f) a CDR-L3 comprising an amino acid sequence selected from SEQ ID NO: 14.

In any of the above embodiments, the anti-RSV antibody is human or humanized. In one embodiment, the anti-RSV antibody comprises CDRs as in any of the above embodiments, and further comprises an acceptor human framework, such as a human immunoglobulin framework or a human consensus framework.

In another aspect, the anti-RSV antibody comprises a heavy chain variable domain (VH) sequence having at least 90%, 91%, 92%, 93%, 94%, 95%, 96%, 97%, 98%, 99% or 100% sequence identity to an amino acid sequence of SEQ ID NO: 15. In certain embodiments, the VH sequence having at least 90%, 91%, 92% 93%, 94%, 95%, 96%, 97%, 98% or 99% identity contains substitutions (e.g., conservative substitutions), insertions or deletions relative to a reference sequence, but the anti-RSV antibody comprising the sequence retains the ability to bind to RSV. In certain embodiments, a total of 1 to 10 amino acids are substituted, inserted and/or deleted in SEQ ID NO: 15. In certain embodiments, the substitutions, insertions or deletions occur in regions other than CDRs (i.e., in FRs). Optionally, the anti-RSV antibody comprises a VH sequence in SEQ ID NO: 15, including a post-translational modification of the sequence. In a particular embodiment, the VH comprises one, two or three CDRs selected from: (a) a CDR-H1 comprising an amino acid sequence of SEQ ID NO: 9, (b) a CDR-H2 comprising an amino acid sequence of SEQ ID NO: 10, and (c) a CDR-H3 comprising an amino acid sequence of SEQ ID NO: 11.

In another aspect, an anti-RSV antibody is provided, wherein the antibody comprises a light chain variable domain (VL) having at least 90%, 91%, 92%, 93%, 94%, 95%, 96%, 97%, 98%, 99% or 100% sequence identity to an amino acid sequence of SEQ ID NO: 16. In certain embodiments, the VL sequence having at least 90%, 91%, 92% 93%, 94%, 95%, 96%, 97%, 98% or 99% identity contains substitutions (e.g., conservative substitutions), insertions or deletions relative to a reference sequence, but the anti-RSV antibody comprising the sequence retains the ability to bind to RSV. In certain embodiments, a total of 1 to 10 amino acids are substituted, inserted and/or deleted in SEQ ID NO: 16. In certain embodiments, the substitutions, insertions or deletions occur in regions other than CDRs (i.e., in FRs). Optionally, the anti-RSV antibody comprises a VL sequence in SEQ ID NO: 16, including a post-translational modification of the sequence. In a particular embodiment, the VL comprises one, two or three CDRs selected from: (a) a CDR-L1 comprising an amino acid sequence of SEQ ID NO: 12; (b) a CDR-L2 comprising an amino acid sequence of SEQ ID NO: 13; and (c) a CDR-L3 comprising an amino acid sequence of SEQ ID NO: 14.

In another aspect, an anti-RSV antibody is provided, wherein the antibody comprises a VH as in any of the embodiments provided above, and a VL as in any of the embodiments provided above. In one embodiment, the antibody comprises a VH sequence in SEQ ID NO: 15 and a VL sequence in SEQ ID NO: 16, respectively, including post-translational modifications of those sequences.

In yet another aspect, the present invention provides an antibody that binds to the same epitope as the anti-RSV antibody provided herein. For example, in certain embodiments, an antibody that binds to the same epitope as an anti-RSV antibody comprising a VH sequence of SEQ ID NO: 15 and a VL sequence of SEQ ID NO: 16 is provided.

In one aspect, the present invention provides an anti-RSV antibody, which comprises at least one, two, three, four, five or six CDRs selected from: (a) a CDR-H1 comprising an amino acid sequence of SEQ ID NO: 17; (b) a CDR-H2 comprising an amino acid sequence of SEQ ID NO: 18; (c) a CDR-H3 comprising an amino acid sequence of SEQ ID NO: 19; (d) a CDR-L1 comprising an amino acid sequence of SEQ ID NO: 20; (e) a CDR-L2 comprising an amino acid sequence of SEQ ID NO: 21; and (f) a CDR-L3 comprising an amino acid sequence of SEQ ID NO: 22.

In one aspect, the present invention provides an antibody, which comprises at least one, at least two or all three VH CDR sequences selected from: (a) a CDR-H1 comprising an amino acid sequence of SEQ ID NO: 17; (b) a CDR-H2 comprising an amino acid sequence of SEQ ID NO: 18; and (c) a CDR-H3 comprising an amino acid sequence of SEQ ID NO: 19. In one embodiment, the antibody comprises a CDR-H3 comprising an amino acid sequence of SEQ ID NO: 19. In another embodiment, the antibody comprises a CDR-H3 comprising an amino acid sequence of SEQ ID NO: 19 and a CDR-L3 comprising an amino acid sequence of SEQ ID NO: 22. In yet another embodiment, the antibody comprises a CDR-H3 comprising an amino acid sequence of SEQ ID NO: 19, a CDR-L3 comprising an amino acid sequence of SEQ ID

**EP 4 588 936 A1**

NO: 22 and a CDR-H2 comprising an amino acid sequence of SEQ ID NO: 18. In yet another embodiment, the antibody comprises (a) a CDR-H1 comprising an amino acid sequence of SEQ ID NO: 17; (b) a CDR-H2 comprising an amino acid sequence of SEQ ID NO: 18; and (c) a CDR-H3 comprising an amino acid sequence of SEQ ID NO: 19.

In another aspect, the present invention provides an antibody, which comprises at least one, at least two or all three VL CDR sequences selected from: (a) a CDR-L1 comprising an amino acid sequence of SEQ ID NO: 20; (b) a CDR-L2 comprising an amino acid sequence of SEQ ID NO: 21; and (c) a CDR-L3 comprising an amino acid sequence of SEQ ID NO: 22. In one embodiment, the antibody comprises (a) a CDR-L1 comprising an amino acid sequence of SEQ ID NO: 20; (b) a CDR-L2 comprising an amino acid sequence of SEQ ID NO: 21; and (c) a CDR-L3 comprising an amino acid sequence of SEQ ID NO: 22.

In another aspect, the antibody of the present invention comprises (a) a VH domain comprising at least one, at least two or all three VH CDR sequences selected from: (i) a CDR-H1 comprising an amino acid sequence of SEQ ID NO: 17, (ii) a CDR-H2 comprising an amino acid sequence of SEQ ID NO: 18, and (iii) a CDR-H3 comprising an amino acid sequence of SEQ ID NO: 19; and (b) a VL domain comprising at least one, at least two or all three VL CDR sequences selected from: (i) a CDR-L1 comprising an amino acid sequence of SEQ ID NO: 20, (ii) a CDR-L2 comprising an amino acid sequence of SEQ ID NO: 21, and (c) a CDR-L3 comprising an amino acid sequence of SEQ ID NO: 22.

In another aspect, the present invention provides an antibody, which comprises (a) a CDR-H1 comprising an amino acid sequence of SEQ ID NO: 17; (b) a CDR-H2 comprising an amino acid sequence of SEQ ID NO: 18; (c) a CDR-H3 comprising an amino acid sequence of SEQ ID NO: 19; (d) a CDR-L1 comprising an amino acid sequence of SEQ ID NO: 20; (e) a CDR-L2 comprising an amino acid sequence of SEQ ID NO: 21; and (f) a CDR-L3 comprising an amino acid sequence selected from SEQ ID NO: 22.

In any of the above embodiments, the anti-RSV antibody is human or humanized. In one embodiment, the anti-RSV antibody comprises CDRs as in any of the above embodiments, and further comprises an acceptor human framework, such as a human immunoglobulin framework or a human consensus framework.

In another aspect, the anti-RSV antibody comprises a heavy chain variable domain (VH) sequence having at least 90%, 91%, 92%, 93%, 94%, 95%, 96%, 97%, 98%, 99% or 100% sequence identity to an amino acid sequence of SEQ ID NO: 23. In certain embodiments, the VH sequence having at least 90%, 91%, 92% 93%, 94%, 95%, 96%, 97%, 98% or 99% identity contains substitutions (e.g., conservative substitutions), insertions or deletions relative to a reference sequence, but the anti-RSV antibody comprising the sequence retains the ability to bind to RSV. In certain embodiments, a total of 1 to 10 amino acids are substituted, inserted and/or deleted in SEQ ID NO: 23. In certain embodiments, the substitutions, insertions or deletions occur in regions other than CDRs (i.e., in FRs). Optionally, the anti-RSV antibody comprises a VH sequence in SEQ ID NO: 23, including a post-translational modification of the sequence. In a particular embodiment, the VH comprises one, two or three CDRs selected from: (a) a CDR-H1 comprising an amino acid sequence of SEQ ID NO: 17, (b) a CDR-H2 comprising an amino acid sequence of SEQ ID NO: 18, and (c) a CDR-H3 comprising an amino acid sequence of SEQ ID NO: 19.

In another aspect, an anti-RSV antibody is provided, wherein the antibody comprises a light chain variable domain (VL) having at least 90%, 91%, 92%, 93%, 94%, 95%, 96%, 97%, 98%, 99% or 100% sequence identity to an amino acid sequence of SEQ ID NO: 24. In certain embodiments, the VL sequence having at least 90%, 91%, 92% 93%, 94%, 95%, 96%, 97%, 98% or 99% identity contains substitutions (e.g., conservative substitutions), insertions or deletions relative to a reference sequence, but the anti-RSV antibody comprising the sequence retains the ability to bind to RSV. In certain embodiments, a total of 1 to 10 amino acids are substituted, inserted and/or deleted in SEQ ID NO: 24. In certain embodiments, the substitutions, insertions or deletions occur in regions other than CDRs (i.e., in FRs). Optionally, the anti-RSV antibody comprises a VL sequence in SEQ ID NO: 24, including a post-translational modification of the sequence. In a particular embodiment, the VL comprises one, two or three CDRs selected from: (a) a CDR-L1 comprising an amino acid sequence of SEQ ID NO: 20; (b) a CDR-L2 comprising an amino acid sequence of SEQ ID NO: 21; and (c) a CDR-L3 comprising an amino acid sequence of SEQ ID NO: 22.

In another aspect, an anti-RSV antibody is provided, wherein the antibody comprises a VH as in any of the embodiments provided above, and a VL as in any of the embodiments provided above. In one embodiment, the antibody comprises a VH sequence in SEQ ID NO: 23 and a VL sequence in SEQ ID NO: 24, respectively, including post-translational modifications of those sequences.

In yet another aspect, the present invention provides an antibody that binds to the same epitope as the anti-RSV antibody provided herein. For example, in certain embodiments, an antibody that binds to the same epitope as an anti-RSV antibody comprising a VH sequence of SEQ ID NO: 23 and a VL sequence of SEQ ID NO: 24 is provided.

In one aspect, the present invention provides an anti-RSV antibody, which comprises at least one, two, three, four, five or six CDRs selected from: (a) a CDR-H1 comprising an amino acid sequence of SEQ ID NO: 25; (b) a CDR-H2 comprising an amino acid sequence of SEQ ID NO: 26; (c) a CDR-H3 comprising an amino acid sequence of SEQ ID NO: 27; (d) a CDR-L1 comprising an amino acid sequence of SEQ ID NO: 28; (e) a CDR-L2 comprising an amino acid sequence of SEQ ID NO: 29; and (f) a CDR-L3 comprising an amino acid sequence of SEQ ID NO: 30.

In one aspect, the present invention provides an antibody, which comprises at least one, at least two or all three VH CDR sequences selected from: (a) a CDR-H1 comprising an amino acid sequence of SEQ ID NO: 25; (b) a CDR-H2 comprising an amino acid sequence of SEQ ID NO: 26; and (c) a CDR-H3 comprising an amino acid sequence of SEQ ID NO: 27. In one embodiment, the antibody comprises a CDR-H3 comprising an amino acid sequence of SEQ ID NO: 27. In another embodiment, the antibody comprises a CDR-H3 comprising an amino acid sequence of SEQ ID NO: 27 and a CDR-L3 comprising an amino acid sequence of SEQ ID NO: 30. In yet another embodiment, the antibody comprises a CDR-H3 comprising an amino acid sequence of SEQ ID NO: 27, a CDR-L3 comprising an amino acid sequence of SEQ ID NO: 30 and a CDR-H2 comprising an amino acid sequence of SEQ ID NO: 26. In yet another embodiment, the antibody comprises (a) a CDR-H1 comprising an amino acid sequence of SEQ ID NO: 25; (b) a CDR-H2 comprising an amino acid sequence of SEQ ID NO: 26; and (c) a CDR-H3 comprising an amino acid sequence of SEQ ID NO: 27.

In another aspect, the present invention provides an antibody, which comprises at least one, at least two or all three VL CDR sequences selected from: (a) a CDR-L1 comprising an amino acid sequence of SEQ ID NO: 28; (b) a CDR-L2 comprising an amino acid sequence of SEQ ID NO: 29; and (c) a CDR-L3 comprising an amino acid sequence of SEQ ID NO: 30. In one embodiment, the antibody comprises (a) a CDR-L1 comprising an amino acid sequence of SEQ ID NO: 28; (b) a CDR-L2 comprising an amino acid sequence of SEQ ID NO: 29; and (c) a CDR-L3 comprising an amino acid sequence of SEQ ID NO: 30.

In another aspect, the antibody of the present invention comprises (a) a VH domain comprising at least one, at least two or all three VH CDR sequences selected from: (i) a CDR-H1 comprising an amino acid sequence of SEQ ID NO: 25, (ii) a CDR-H2 comprising an amino acid sequence of SEQ ID NO: 26, and (iii) a CDR-H3 comprising an amino acid sequence of SEQ ID NO: 27; and (b) a VL domain comprising at least one, at least two or all three VL CDR sequences selected from: (i) a CDR-L1 comprising an amino acid sequence of SEQ ID NO: 28, (ii) a CDR-L2 comprising an amino acid sequence of SEQ ID NO: 29, and (c) a CDR-L3 comprising an amino acid sequence of SEQ ID NO: 30.

In another aspect, the present invention provides an antibody, which comprises (a) a CDR-H1 comprising an amino acid sequence of SEQ ID NO: 25; (b) a CDR-H2 comprising an amino acid sequence of SEQ ID NO: 26; (c) a CDR-H3 comprising an amino acid sequence of SEQ ID NO: 27; (d) a CDR-L1 comprising an amino acid sequence of SEQ ID NO: 28; (e) a CDR-L2 comprising an amino acid sequence of SEQ ID NO: 29; and (f) a CDR-L3 comprising an amino acid sequence selected from SEQ ID NO: 30.

In any of the above embodiments, the anti-RSV antibody is human or humanized. In one embodiment, the anti-RSV antibody comprises CDRs as in any of the above embodiments, and further comprises an acceptor human framework, such as a human immunoglobulin framework or a human consensus framework.

In another aspect, the anti-RSV antibody comprises a heavy chain variable domain (VH) sequence having at least 90%, 91%, 92%, 93%, 94%, 95%, 96%, 97%, 98%, 99% or 100% sequence identity to an amino acid sequence of SEQ ID NO: 31. In certain embodiments, the VH sequence having at least 90%, 91%, 92% 93%, 94%, 95%, 96%, 97%, 98% or 99% identity contains substitutions (e.g., conservative substitutions), insertions or deletions relative to a reference sequence, but the anti-RSV antibody comprising the sequence retains the ability to bind to RSV. In certain embodiments, a total of 1 to 10 amino acids are substituted, inserted and/or deleted in SEQ ID NO: 31. In certain embodiments, the substitutions, insertions or deletions occur in regions other than CDRs (i.e., in FRs). Optionally, the anti-RSV antibody comprises a VH sequence in SEQ ID NO: 31, including a post-translational modification of the sequence. In a particular embodiment, the VH comprises one, two or three CDRs selected from: (a) a CDR-H1 comprising an amino acid sequence of SEQ ID NO: 25, (b) a CDR-H2 comprising an amino acid sequence of SEQ ID NO: 26, and (c) a CDR-H3 comprising an amino acid sequence of SEQ ID NO: 27.

In another aspect, an anti-RSV antibody is provided, wherein the antibody comprises a light chain variable domain (VL) having at least 90%, 91%, 92%, 93%, 94%, 95%, 96%, 97%, 98%, 99% or 100% sequence identity to an amino acid sequence of SEQ ID NO: 32. In certain embodiments, the VL sequence having at least 90%, 91%, 92% 93%, 94%, 95%, 96%, 97%, 98% or 99% identity contains substitutions (e.g., conservative substitutions), insertions or deletions relative to a reference sequence, but the anti-RSV antibody comprising the sequence retains the ability to bind to RSV. In certain embodiments, a total of 1 to 10 amino acids are substituted, inserted and/or deleted in SEQ ID NO: 32. In certain embodiments, the substitutions, insertions or deletions occur in regions other than CDRs (i.e., in FRs). Optionally, the anti-RSV antibody comprises a VL sequence in SEQ ID NO: 32, including a post-translational modification of the sequence. In a particular embodiment, the VL comprises one, two or three CDRs selected from: (a) a CDR-L1 comprising an amino acid sequence of SEQ ID NO: 28, (b) a CDR-L2 comprising an amino acid sequence of SEQ ID NO: 29, and (c) a CDR-L3 comprising an amino acid sequence of SEQ ID NO: 30.

In another aspect, an anti-RSV antibody is provided, wherein the antibody comprises a VH as in any of the embodiments provided above, and a VL as in any of the embodiments provided above. In one embodiment, the antibody comprises a VH sequence in SEQ ID NO: 31 and a VL sequence in SEQ ID NO: 32, respectively, including post-translational modifications of those sequences.

In yet another aspect, the present invention provides an antibody that binds to the same epitope as the anti-RSV antibody provided herein. For example, in certain embodiments, an antibody that binds to the same epitope as an anti-RSV antibody comprising a VH sequence of SEQ ID NO: 31 and a VL sequence of SEQ ID NO: 32 is provided.

In yet another aspect of the present invention, the anti-RSV antibody according to any of the above embodiments is a monoclonal antibody, including a chimeric, humanized or human antibody. In one embodiment, the anti-RSV antibody is an antibody fragment, e.g., an Fv, Fab, Fab', xFab, scFv, diabody or F(ab')$_2$ fragment. In another embodiment, the antibody is a full-length antibody, e.g., having substitutions L234A, L235A and P329G (LALA-PG) in the Fc region derived from the human IgG1 Fc region (see e.g., WO 2012/130831 A1).

In yet another aspect, the anti-RSV antibody according to any of the above embodiments may incorporate any of the features, singly or in combination, described in Sections 1-7 below:

## 1. Antibody affinity

In certain embodiments, the antibody provided herein has a dissociation constant (Kd) of $\leq 1$ $\mu$M, $\leq 100$ nM, $\leq 10$ nM, $\leq 1$ nM, $\leq 0.1$ nM, $\leq 0.01$ nM or $\leq 0.001$ nM (e.g., $10^{-8}$ M or less, e.g., $10^{-8}$ M to $10^{-13}$ M, e.g., $10^{-9}$ M to $10^{-13}$ M).

In one embodiment, Kd is measured by a radiolabeled antigen binding assay (RIA). In one embodiment, RIA is performed with the Fab version of an antibody of interest and its antigen. For example, the solution binding affinity of Fabs for antigen is measured by equilibrating the Fab using a minimal concentration of ($^{125}$I) labeled antigen in the presence of a titration series of unlabeled antigens, and then capturing the bound antigens using a plate coated with an anti-Fab antibody (see, e.g., Chen et al., J. Mol. Biol. 293: 865-881 (1999)). To establish conditions for the assay, a MICROTITER® multi-well plate (Thermo Scientific) is coated overnight with 5 $\mu$g/ml capture anti-Fab antibody (Cappel Labs) in 50 mM sodium carbonate (pH 9.6), and then blocked with 2% (w/v) bovine serum albumin in PBS for 2-5 hours at room temperature (about 23°C). In a non-adsorbent plate (Nunc #269620), 100 pM or 26 pM [$^{125}$I]-antigen is mixed with a serially diluted Fab of interest (e.g., consistent with the evaluation of anti-VEGF antibody, Fab-12, in Presta et al., Cancer Res. 57: 4593-4599 (1997)). The Fab of interest is then incubated overnight; however, the incubation may continue for a longer period (e.g., about 65 hours) to ensure that the equilibrium is reached. Thereafter, the mixture is transferred to the capture plate for incubation at room temperature (for example, 1 hour). The solution is then removed and the plate is washed 8 times with 0.1% polysorbate 20 (TWEEN-20®) in PBS. After the plate is dried, a scintillation solution (MICROSCINT-20™; Packard) is added at 150 $\mu$l/well and the plate is counted on a TOPCOUNT™ gamma counter (Packard) for 10 minutes. The concentration at which each Fab gives less than or equal to 20% of maximal binding is selected for use in competitive binding assay.

According to another embodiment, Kd is measured using a BIACORE® surface plasmon resonance assay. For example, the assay is performed using BIACORE®-2000 or BIACORE®-3000 (BIAcore, Inc., Piscataway, NJ) with an immobilized antigen CM5 chip at about 10 response units (RUs) at 25°C. In one embodiment, a carboxymethylated dextran biosensor chip (CM5, BIACORE, Inc.) is activated with N-ethyl-N-(3-dimethylaminopropyl)-carbodiimide hydrochloride (EDC) and N-hydroxysuccinimide (NHS) according to the supplier's instructions. An antigen is diluted to 5 $\mu$g/ml (about 0.2 $\mu$M) with 10 mM sodium acetate pH 4.8, and then injected at a flow rate of 5 $\mu$l/min to obtain about 10 response units (RUs) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of about 25 $\mu$l/min. The association rate ($k_{on}$) and the dissociation rate ($k_{off}$) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio $k_{off}/k_{on}$. See, e.g., Chen et al., J. Mol. Biol. 293: 865-881 (1999). If the association rate exceeds $10^6$ M$^{-1}$ S$^{-1}$ according to the above surface plasmon resonance assay, the association rate can be determined using a fluorescence quenching technique, i.e., measuring the increase or decrease in fluorescence emission intensity (excitation = 295 nm; emission = 340 nm, 16 nm bandpass) at 25°C of a 20 nM anti-antigen antibody (Fab form) in PBS pH 7.2, in the presence of increasing concentrations of the antigen, as measured in a spectrometer, such as a stop-flow equipped spectrophometer (Aviv Instruments) or a 8000-series SLM-AMINCO™ spectrophotometer (ThermoSpectronic) with a stirred cuvette.

## 2. Antibody fragments

In certain embodiments, the antibody provided herein is an antibody fragment. The term "antibody fragment" refers to a molecule other than an intact antibody that comprises a portion of an intact antibody that retains the ability to specifically bind to an antigen. Antibody fragments include, but are not limited to, Fab, Fab', Fab'-SH, F(ab')$_2$, xFab, Fv, single-chain Fab (scFab), single-chain variable fragments (scFvs) and single-domain antibodies (dAbs). For a review of certain antibody fragments, see Holliger and Hudson, Nature Biotechnology 23: 1126-1136 (2005).

In one embodiment, the antibody fragment is a Fab, Fab', Fab'-SH, xFab, or F(ab')$_2$ fragment, particularly a Fab fragment. An intact antibody is digested with papain to produce two identical antigen-binding fragments, referred to as "Fab" fragments, each containing a variable domain of heavy chain and a variable domain of light chain, and also a constant domain of the light chain and the first constant domain (CH1) of the heavy chain. Thus, the term "Fab fragment" refers to an antibody fragment comprising the VL domain and the constant domain (CL) of a light chain, and the VH domain and the first constant domain (CH1) of a heavy chain. The term "xFab fragment" refers to a Fab fragment in which the VH domain is swapped with the VL or the CH1 domain is swapped with the CL domain. The Fab' fragment differs from the Fab fragment by adding residues at the carboxyl terminus of the CH1 domain of the heavy chain, including one or more cysteines from the hinge region of the antibody. Fab'-SH is a Fab' fragment in which the cysteine residue of the constant domain has a free sulfhydryl group. Treatment with pepsin produces a $F(ab')_2$ fragment having two antigen-binding sites (two Fab fragments) and a portion of the Fc region. For a discussion of Fab and $F(ab')_2$ fragments comprising salvage receptor binding epitope residues and having increased in vivo half-life, see U. S. Patent No. 5,869,046.

In another embodiment, the antibody fragment is a diabody, triabody, or tetrabody. Diabodies are antibody fragments having two antigen-binding sites, which may be bivalent or bispecific. See, e.g., EP 404, 097; WO 1993/01161; Hudson et al., Nat. Med. 9: 129-134 (2003); and Hollinger et al., Proc. Natl. Acad. Sci. USA 90: 6444-6448 (1993). Tribodies and tetrabodies are also recorded in Hudson et al., Nat. Med. 9: 129-134 (2003).

In yet another embodiment, the antibody fragment is a single-chain Fab fragment. A "single-chain Fab fragment" or "scFab" is a polypeptide consisting of an antibody heavy chain variable domain (VH), an antibody constant domain 1 (CH1), an antibody light chain variable domain (VL), an antibody light chain constant domain (CL), and a linker, wherein the antibody domains and the linker have one of the following orders in a direction from N- to C-terminus: a) VH-CH1-linker-VL-CL, b) VL-CL-linker-VH-CH1, c) VH-CL-linker-VL-CH1 or d) VL-CH1-linker-VH-CL. In particular, the linker is a polypeptide having at least 30 amino acids, preferably between 32 and 50 amino acids. The single-chain Fab fragment is stabilized via a natural disulfide bond between the CL domain and the CH1 domain. In addition, these single-chain Fab molecules can be further stabilized via interchain disulfide bonds created by the insertion of cysteine residues (e.g., position 44 in the variable heavy chain and position 100 in the variable light chain according to Kabat numbering).

In another embodiment, the antibody fragment is a single-chain variable fragment (scFv). A "single-chain variable fragment (scFv)" is a fusion protein of a heavy chain variable region ($V_H$) and a light chain variable region ($V_L$) of an antibody linked via a linker. In particular, the linker is a short polypeptide of 10 to 25 amino acids and usually rich in glycine for flexibility and serine or threonine for solubility, and can link the N-terminus of $V_H$ to the C-terminus of $V_L$, or vice versa. This protein retains the specificity of the original antibody despite the removal of the constant regions and the introduction of a linker. For a review of scFv fragments, see, e.g., Pluckthün, in The Pharmacology of Monoclonal Antibodies, vol. 113, Rosenburg and Moore, eds., (Springer-Verlag, New York), pages 269-315 (1994); see also WO 93/16185; and U. S. Patent Nos. 5,571,894 and 5,587458.

In another embodiment, the antibody fragment is a single-domain antibody. Single-domain antibodies are antibody fragments that comprise all or a portion of the heavy chain variable domain or all or a portion of the light chain variable domain of an antibody. In certain embodiments, the single-domain antibody is a human single-domain antibody (Domantis, Inc., Waltham, MA; see, e.g., U. S. Patent No. 6,248,516 B1).

Antibody fragments can be made by a variety of techniques, including, but not limited to, proteolytic digestion of intact antibodies and generation by recombinant host cells, such as *E. coli* or phage, as described herein.

### 3. Chimeric and humanized antibodies

In certain embodiments, the antibody provided herein is a chimeric antibody. Certain chimeric antibodies are recorded, for example, in U. S. Patent No. 4,816,567; and Morrison et al., Proc. Natl. Acad. Sci. USA, 81: 6851-6855 (1984). In one example, the chimeric antibody comprises a non-human variable region (e.g., a variable region derived from a mouse, rat, hamster, rabbit, or non-human primate, such as a monkey) and a human constant region. In yet another example, a chimeric antibody is a "class switched" antibody in which the class or subclass has been changed from that of the parent antibody. Chimeric antibodies include antigen-binding fragments thereof.

In certain embodiments, the chimeric antibody is a humanized antibody. Generally, non-human antibodies are humanized to reduce immunogenicity to humans while retaining the specificity and affinity of the parent non-human antibody. In general, a humanized antibody comprises one or more variable domains, in which HVRs, e.g., CDRs (or portions thereof), are derived from a non-human antibody, and FRs (or portions thereof) are derived from human antibody sequences. Optionally, a humanized antibody will also comprise at least a portion of a human constant region. In some embodiments, some FR residues in a humanized antibody are substituted with corresponding residues from a non-human antibody (e.g., the antibody from which HVR residues are derived), e.g., to restore or improve antibody specificity or affinity.

Humanized antibodies and methods of making them are reviewed, for example, in Almagro and Fransson, Front. Biosci. 13: 1619-1633 (2008), and are further recorded for example, in Riechmann et al., Nature 332: 323-329 (1988); Queen et al., Proc. Nat'l Acad. Sci. USA 86: 10029-10033 (1989); U. S. Patent Nos. 5,821,337, 7,527,791, 6,982,321 and 7,087,409; Kashmiri et al., Methods 36: 25-34 (2005) (describing specificity determinant region (SDR) grafting); Padlan, Mol. Immunol. 28: 489-498 (1991) (describing "resurfacing"); Dall'Acqua et al., Methods 36: 43-60 (2005) (describing "FR shuffling"); and Osbourn et al., Methods 36: 61-68 (2005) and Klimka et al., Br. J. Cancer 83: 252-260 (2000) (describing the "guided selection" approach to FR shuffling).

Human framework regions that can be used for humanization include, but are not limited to, framework regions selected using the "best-fit" method (see, e.g., Sims et al., J. Immunol. 151: 2296 (1993)); framework regions derived from the consensus sequence of human antibodies of a particular subgroup of light or heavy chain variable regions (see, e.g., Carter et al., Proc. Natl. Acad. Sci. USA, 89: 4285 (1992); and Presta et al., J. Immunol., 151: 2623 (1993)); human mature (somatically mutated) framework regions or human germline framework regions (see, e.g., Almagro and Fransson, Front. Biosci. 13: 1619-1633 (2008)); and framework regions derived by screening FR libraries (see, e.g., Baca et al., J. Biol. Chem. 272: 10678-10684 (1997) and Rosok et al., J. Biol. Chem. 271: 22611-22618 (1996)).

### 4. Human antibodies

In certain embodiments, the antibody provided herein is a human antibody. Human antibodies can be generated using a variety of techniques known in the art. Human antibodies are recorded generally in van Dijk and van de Winkel, Curr. Opin. Pharmacol. 5: 368-74 (2001) and Lonberg, Curr. Opin. Immunol. 20: 450-459 (2008).

Human antibodies can be prepared by administering immunogens to transgenic animals that have been modified to generate intact human antibodies or intact antibodies with human variable regions in response to antigenic challenge. Such animals usually contain all or a portion of the human immunoglobulin loci, which replace the endogenous immunoglobulin loci, or which are present extrachromosomally or are randomly integrated into the chromosomes of the animal. In such transgenic mice, the endogenous immunoglobulin loci have generally been inactivated. For a review of methods for obtaining human antibodies from transgenic animals, see Lonberg, Nat. Biotech. 23: 1117-1125 (2005). See also, e.g., U. S. Patent Nos. 6,075,181 and 6,150,584 describing XENOMOUSE™ technology; U. S. Patent No. 5,770,429 describing HUMAB® technology; U. S. Patent No. 7,041,870 describing K-M MOUSE® technology, and U. S. Patent Application Publication No. US 2007/0061900 describing VELOCIMOUSE® technology). Human variable regions from intact antibodies generated by such animals may be further modified, e.g., by combining with a different human constant region.

Human antibodies can also be generated by hybridoma-based methods. Human myeloma and mouse-human heteromyeloma cell lines for the generation of human monoclonal antibodies have been recorded (see, e.g., Kozbor, J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991)). Human antibodies generated by human B cell hybridoma technology are also recorded in Li et al., Proc. Natl. Acad. Sci. USA, 103: 3557-3562 (2006). Additional methods include those recorded, for example, in U. S. Patent No. 7,189,826 (describing generation of monoclonal human IgM antibodies from hybridoma cell lines) and Ni, Xiandai Mianyixue, 26(4): 265-268 (2006) (describing human-human hybridomas). Human hybridoma technology (Trioma technology) is also recorded in Vollmers and Brandlein, Histology and Histopathology, 20(3): 927-937 (2005) and Vollmers and Brandlein, Methods and Findings in Experimental and Clinical Pharmacology, 27(3): 185-91 (2005).

Human antibodies can also be generated by isolating Fv clone variable domain sequences selected from human-derived phage display libraries. Such variable domain sequences can then be combined with a desired human constant domain. Techniques for selecting human antibodies from antibody libraries are described below.

### 5. Library-derived antibodies

The antibody of the present invention can be isolated by screening combinatorial libraries for antibodies having one or more desired activities. Methods for screening combinatorial libraries are reviewed, for example, in Lerner et al., Nature Reviews 16: 498-508 (2016). For example, a variety of methods are known in the art for generating phage display libraries and screening such libraries for antibodies possessing the desired binding characteristics. Such methods are reviewed, for example, in Frenzel et al., mAbs 8: 1177-1194 (2016); Bazan et al., Human Vaccines and Immunotherapeutics 8: 1817-1828 (2012); and Zhao et al., Critical Reviews in Biotechnology 36: 276-289 (2016) and Hoogenboom et al., in Methods in Molecular Biology 178: 1-37 (O'Brien et al., eds., Human Press, Totowa, NJ, 2001) and Marks and Bradbury, in Methods in Molecular Biology 248: 161-175 (Lo, ed., Human Press, Totowa, NJ, 2003).

In certain phage display methods, repertoires of VH and VL genes are cloned separately by polymerase chain reaction (PCR) and randomly recombined in phage libraries, which can then be screened for antigen-binding phages, as recorded in Winter et al., Annual Review of Immunology 12: 433-455 (1994). Phages generally display antibody fragments, either as single-chain Fv (scFv) fragments or as Fab fragments. Libraries from immunized sources provide high-affinity antibodies to the immunogens without the requirement of constructing hybridomas. Alternatively, a naive repertoire can be cloned (e.g., from humans) to provide a single source of antibodies to a wide range of non-self and also self antigens without any immunization, as recorded by Griffiths et al., EMBO Journal 12: 725-734 (1993). Finally, naive libraries can also be synthetically generated by cloning unrearranged V gene segments from stem cells, and using PCR primers containing random sequences to encode the highly variable CDR3 regions and to accomplish rearrangement *in vitro,* as recorded by Hoogenboom and Winter, Journal of Molecular Biology 227: 381-388 (1992). Patent publications describing human antibody phage libraries include, for example, U. S. Patent Nos. 5,750,373; 7,985,840; 7,785,903 and 8,679,490 and U. S. Patent Publication Nos. 2005/0079574, 2007/0117126, 2007/0237764 and 2007/0292936.

Further examples of methods known in the art for screening combinatorial libraries for antibodies having one or more desired activities include ribosome and mRNA displays, and methods for displaying and selecting antibodies on bacteria, mammalian cells, insect cells, or yeast cells. For a review of methods used for yeast surface display, see, e.g., Scholler et al., Methods in Molecular Biology 503: 135-56 (2012) and Cherf et al., Methods in Molecular biology 1319: 155-175 (2015) and Zhao et al., Methods in Molecular Biology 889: 73-84 (2012). Methods for ribosome display are recorded, for example, in He et al., Nucleic Acids Research 25: 5132-5134 (1997) and Hanes et al., PNAS 94: 4937-4942 (1997).

Antibodies or antibody fragments isolated from human antibody libraries are considered human antibodies or human antibody fragments herein.

### 6. Multispecific antibodies

In certain embodiments, the antibody provided herein is a multispecific antibody, e.g., a bispecific antibody. Multispecific antibodies are monoclonal antibodies that have binding specificities for at least two different sites (i.e., different epitopes on different antigens or different epitopes on the same antigen). In certain embodiments, the multispecific antibody has three or more binding specificities. In certain embodiments, one of the binding specificities is for RSV, while the other (two or more) specificities are for any other antigen. In certain embodiments, the bispecific antibody can bind two (or more) different antigens or epitopes of RSV. Multispecific (e.g., bispecific) antibodies may also be used to localize cytotoxic agents or cells to cells infected with RSV. Multispecific antibodies can be prepared as full-length antibodies or antibody fragments.

Techniques for generating multispecific antibodies include, but are not limited to, recombinant co-expression of two immunoglobulin heavy chain-light chain pairs having different specificities (see Milstein and Cuello, Nature 305: 537 (1983)), and "knob-in-hole" engineering (see, e.g., U. S. Patent No. 5,731,168 and Atwell et al., J. Mol. Biol. 270: 26 (1997)). Multispecific antibodies may also be generated by the engineering electrostatic steering effect for producing antibody Fc-heterodimer molecules (see, e.g., WO 2009/089004); cross-linking two or more antibodies or fragments (see, e.g., U. S. Patent No. 4,676,980, and Brennan et al., Science, 229: 81 (1985)); using leucine zippers to generate bispecific antibodies (see, e.g., Kostelny et al., J. Immunol., 148(5): 1547-1553 (1992) and WO 2011/034605); using common light chain technology to circumvent light chain mispairing problem (see, e.g., WO 98/50431); using "diabody" technology for generating bispecific antibody fragments (see, e.g., Hollinger et al., Proc. Natl. Acad. Sci. USA, 90: 6444-6448 (1993)); and using single-chain Fv (sFv) dimers (see, e.g., Gruber et al., J. Immunol., 152: 5368 (1994)); and preparing trispecific antibodies as recorded, for example, in Tutt et al., J. Immunol. 147: 60 (1991).

Engineered antibodies having three or more antigen-binding sites, including, for example, "octopus antibodies" or DVD-Ig, are also included herein (see, e.g., WO 2001/77342 and WO 2008/024715). Further examples of multispecific antibodies having three or more antigen-binding sites can be found in WO 2010/115589, WO 2010/112193, WO 2010/136172, WO 2010/145792 and WO 2013/026831. Bispecific antibodies or antigen-binding fragments thereof also include "dual-acting FAb" or "DAF" comprising an antigen-binding site that binds to RSV and another different antigen, or two different epitopes of RSV (see, e.g., US 2008/0069820 and WO 2015/095539).

Multispecific antibodies can also be provided in an asymmetric form, with domain swapping in one or more binding arms having the same antigen specificity, i.e., by swapping VH/VL domains (see, e.g., WO 2009/080252 and WO 2015/150447), CH1/CL domains (see, e.g., WO 2009/080253) or the entire Fab arm (see, e.g., WO 2009/080251, WO 2016/016299, see also Schaefer et al., PNAS, 108 (2011) 1187-1191, and Klein et al., MAbs 8 (2016) 1010-20). In one embodiment, the multispecific antibody comprises a cross-Fab fragment. The term **"cross-Fab fragment"** or "xFab fragment" or "domain-swapped Fab fragment" refers to a Fab fragment in which the variable or constant regions of the heavy and light chains are swapped. A cross-Fab fragment comprises a polypeptide chain consisting of a light chain variable region (VL) and a heavy chain constant region (CH1), and a polypeptide chain consisting of a heavy chain variable region (VH) and a light chain constant region (CL). Asymmetric Fab arms can also be engineered by introducing charged or uncharged amino acid mutations into the domain interface to direct correct Fab pairing. See, e.g., WO 2016/172485.

Various additional molecular versions of multispecific antibodies are known in the art and are included herein (see, e.g., Spiess et al., Mol Immunol 67 (2015) 95-106).

### 7. Antibody variants

In certain embodiments, amino acid sequence variants of the antibody provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the antibody. Any combination of deletions, insertions, and substitutions may be made to arrive at the final construct, provided that the final construct possesses the desired characteristics, for example, antigen binding.

**a) Substitution, insertion, and deletion variants**

In certain embodiments, antibody variants having one or more amino acid substitutions are provided. Sites of interest for substitutional mutagenesis include HVRs and FRs. Conservative substitutions are as shown in Table A under the heading of "Preferred substitution". More substantial changes are provided in Table A under the heading of "Exemplary substitution" and as further described below in reference to amino acid side chain classes. Amino acid substitutions can be introduced into an antibody of interest and the products are screened for desired activity, e.g., retained/improved antigen binding, decreased immunogenicity, or improved ADCC or CDC.

**Table A**

| Initial residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

Amino acids may be grouped according to common side-chain properties:

(1) Hydrophobic: norleucine, Met, Ala, Val, Leu, Ile;
(2) Neutral, hydrophilic: Cys, Ser, Thr, Asn, Gln;
(3) Acidic: Asp, Glu;
(4) Basic: His, Lys, Arg;
(5) Residues that affect chain orientation: Gly, Pro;
(6) Aromatic: Trp, Tyr, Phe.

Non-conservative substitutions will entail exchanging a member of one of these classes for another class.
One type of substitutional variant involves substituting one or more hypervariable region residues of a parent antibody, such as a humanized or human antibody. Generally, the resulting variants selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity-matured antibody, which can be conveniently generated, for example, using phage display-based affinity maturation techniques such as those described herein. Briefly, one or more HVR residues are mutated and the variant antibodies are displayed on phage and screened for a particular biological activity (e.g. binding affinity).

Alterations (e.g., substitutions) can be made in HVRs, for example, to improve antibody affinity. Such alterations can be made in HVR "hot spots", i.e., residues encoded by codons that undergo mutations at high frequency during the somatic maturation process (see, e.g., Chowdhury, Methods Mol. Biol. 207: 179-196 (2008)), and/or residues that contact antigen, with the resulting variant VH or VL being tested for binding affinity. Affinity maturation by constructing and reselecting from secondary libraries has been recorded, for example, in Hoogenboom et al., in Methods in Molecular Biology 178: 1-37 (O'Brien et al., eds., Human Press, Totowa, NJ, (2001)). In some embodiments of affinity maturation, diversity is introduced into the variable genes selected for maturation by any one of a variety of methods, such as error-prone PCR, strand shuffling, or oligonucleotide-directed mutagenesis. A secondary library is then created. The library is then screened to identify any antibody variants having the desired affinity. Another method to introduce diversity involves HVR-directed approaches in which several HVR residues (e.g., 4-6 residues at a time) are randomized. HVR residues involved in antigen binding can be specifically identified, for example, using alanine scanning mutagenesis or modeling. In particular, CDR-H3 and CDR-L3 are often targeted.

In certain embodiments, substitutions, insertions, or deletions may occur within one or more HVRs, as long as such alterations do not substantially reduce the ability of an antibody to bind to an antigen. For example, conservative alterations (e.g., conservative substitutions as provided herein) that do not substantially reduce binding affinity can be made in HVRs. Such alterations may, for example, be outside of the antigen contact residues in the HVRs. In certain embodiments of the variant VH and VL sequences provided above, each HVR either is unaltered, or contains no more than 1, 2, or 3 amino acid substitutions.

A useful method for identifying residues or regions of an antibody that may be targeted for mutagenesis is referred to as "alanine scanning mutagenesis", as recorded by Cunningham and Wells (1989) Science, 244: 1081-1085. In this method, a residue or a group of target residues (e.g., charged residues, such as Arg, Asp, His, Lys, and Glu) are identified and replaced by neutral or negatively charged amino acids (such as alanine or polyalanine) to determine whether the interaction of the antibody with the antigen is affected. Further substitutions may be introduced at amino acid positions demonstrating functional sensitivity to initial substitutions. Alternatively/additionally, a crystal structure of an antigen-antibody complex is used to identify the contact point between the antibody and the antigen. Such contact residues and neighboring residues may be targeted or eliminated as candidates for substitution. Variants can be screened to determine whether they possess the desired properties.

Amino acid sequence insertions include amino- and/or carboxyl-terminal fusions ranging in length from one residue to polypeptides containing 100 or more residues, as well as intra-sequence insertions of single or multiple amino acid residues. Examples of terminal insertions include an antibody with an N-terminal methionyl residue. Other insertional variants of antibody molecules include fusions to the N- or C-terminus of an antibody to an enzyme (e.g., for ADEPT) or a polypeptide that increases the serum half-life of the antibody.

### b) Glycosylation variants

In certain embodiments, the antibody provided herein is altered to increase or decrease the extent to which the antibody is glycosylated. Addition or deletion of glycosylation sites to an antibody can be conveniently accomplished by altering the amino acid sequence to create or remove one or more glycosylation sites.

Where the antibody comprises an Fc region, the oligosaccharide attached thereto may be altered. Natural antibodies generated by mammalian cells usually comprise a branched, biantennary oligosaccharide that is usually attached to Asn297 of the CH2 domain of the Fc region via N-linkage. See, Wright et al., TIBTECH 15: 26-32 (1997). The oligosaccharide may include various carbohydrates, such as mannose, N-acetylglucosamine (GlcNAc), galactose, and sialic acid, as well as fucose attached to a GlcNAc in the "stem" of the biantennary oligosaccharide structure. In some embodiments, modifications of the oligosaccharide in the antibody of the present invention can be made in order to create antibody variants having certain improved properties.

In one embodiment, antibody variants are provided that have a non-fucosylated oligosaccharide, i.e., an oligosaccharide structure that lacks fucose, attached (directly or indirectly) to an Fc region. Such non-fucosylated oligosaccharides (also referred to as "afucosylated" oligosaccharides) are particularly N-linked oligosaccharides that lack a fucose residue attached to the first GlcNAc in the stem of the biantennary oligosaccharide structure. In one embodiment, antibody variants are provided that have an increased proportion of non-fucosylated oligosaccharides in the Fc region compared to a natural or parent antibody. For example, the proportion of non-fucosylated oligosaccharides may be at least about 20%, at least about 40%, at least about 60%, at least about 80%, or even about 100% (i.e., no fucosylated oligosaccharides are present). The percentage of non-fucosylated oligosaccharides is the (average) amount of oligosaccharides lacking a fucose residue relative to the sum of all oligosaccharides attached to Asn297 (e.g., complex, hybrid, and high mannose structures), as measured by MALDI-TOF mass spectrometry, for example, as recorded in WO 2006/082515. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such antibodies having an increased proportion of non-fucosylated oligosaccharides in the Fc region may have improved FcγIIIa receptor binding and/or improved effector function, particularly improved ADCC function. See, e.g., US 2003/0157108; US 2004/0093621.

Examples of cell lines capable of generating antibodies having reduced fucosylation include Lec13 CHO cells deficient in protein fucosylation (Ripka et al., Arch. Biochem. Biophys. 249: 533-545 (1986); US 2003/0157108; and WO 2004/056312, especially in Example 11), and knockout cell lines, such as α-1,6-fucosyltransferase gene *FUT8* knockout CHO cells (see, e.g., Yamane-Ohnuki et al., Biotech. Bioeng. 87: 614-622 (2004); Kanda, Y. et al., Biotechnol. Bioeng., 94(4): 680-688 (2006); and WO 2003/085107), or cells with reduced or eliminated GDP-fucose synthesis or transporter activity (see, e.g., US 2004259150, US 2005031613, US 2004132140, US 2004110282).

In yet another embodiment, antibody variants are provided with bisected oligosaccharides, such as biantennary oligosaccharides attached to the Fc region of the antibody, which are bisected by GlcNAc. Such antibody variants may have reduced fucosylation and/or improved ADCC function as described above. Examples of such antibody variants are recorded, for example, in Umana et al., Nat Biotechnol 17, 176-180 (1999); Ferrara et al., Biotechn Bioeng 93, 851-861 (2006); WO 99/54342; WO 2004/065540, WO 2003/011878.

Antibody variants having at least one galactose residue in the oligosaccharide attached to the Fc region are also provided. Such antibody variants may have improved CDC function. Such antibody variants are recorded, for example, in WO 1997/30087; WO 1998/58964; and WO 1999/22764.

### c) <u>Fe region variants</u>

In certain embodiments, one or more amino acid modifications may be introduced into the Fc region of the antibody provided herein, thereby generating an Fc region variant. The Fc region variant may comprise a human Fc region sequence (e.g., a human IgG1, IgG2, IgG3 or IgG4 Fc region) that comprises an amino acid modification (e.g., substitution) at one or more amino acid positions.

In certain embodiments, the present invention encompasses antibody variants that possess some but not all effector functions, which make them desirable candidates for applications where the *in-vivo* half-life of the antibody is important, whereas certain effector functions, such as complement and ADCC, are unnecessary or deleterious. *In-vitro* and/or *in-vivo* cytotoxicity assays can be performed to confirm reduction/depletion of CDC and/or ADCC activity. For example, Fc receptor (FcR) binding assays can be performed to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9: 457-492 (1991). Non-limiting examples of *in-vitro* assays for evaluating ADCC activity of a molecule of interest are recorded in U. S. Patent No. 5,500,362 (see, e.g., Hellstrom, I. et al., Proc. Nat'l Acad. Sci. USA 83: 7059-7063 (1986)) and Hellstrom, I. et al., Proc. Nat'l Acad. Sci. USA 82: 1499-1502 (1985); 5,821,337 (see Bruggemann, M. et al., J. Exp. Med. 166: 1351-1361 (1987)). Alternatively, non-radioactive assays may be employed (see, e.g., ACTI™ non-radioactive cytotoxicity assays for flow cytometry (Cell-Technology, Inc. Mountain View, CA; and CytoTox 96® non-radioactive cytotoxicity assay (Promega Madison, WI)). Useful effector cells for such assays include peripheral blood mononuclear cells (PBMC) and natural killer (NK) cells. Alternatively/additionally, ADCC activity of a molecule of interest can be evaluated *in vivo,* e.g., in animal models such as those disclosed in Clynes et al., Proc. Nat'l Acad. Sci. USA 95: 652-656 (1998). C1q binding assays may also be performed to confirm that the antibody is unable to bind to C1q and therefore lacks CDC activity. See, e.g., C1q and C3c binding ELISA in WO 2006/029879 and WO 2005/100402. To evaluate complement activation, CDC assays may be performed (see, e.g., Gazzano-Santoro et al., J. Immunol. Methods 202: 163 (1996); Cragg, M.S. et al., Blood 101: 1045-1052 (2003); and Cragg, M.S. and M.J. Glennie, Blood 103: 2738-2743 (2004)). FcRn binding and *in-vivo* clearance/half-life assays can also be performed using methods known in the art (see, e.g., Petkova, S.B. et al., Int'l. Immunol. 18(12): 1759-1769 (2006); WO 2013/120929 A1).

Antibodies having reduced effector function include those having substitution of one or more of Fc region residues 238, 265, 269, 270, 297, 327 and 329 (U.S. Patent No. 6,737,056). Such Fc mutants include Fc mutants having substitutions at two or more of amino acid positions 265, 269, 270, 297 and 327, including the so-called "DANA" Fc mutant having substitution of residues 265 and 297 to alanine (U.S. Patent No. 7,332,581).

Certain antibody variants having improved or reduced binding to FcR are described (see, e.g., U. S. Patent No. 6,737,056; WO 2004/056312, and Shields et al., J. Biol. Chem. 9(2): 6591-6604 (2001)).

In certain embodiments, the antibody variant comprises an Fc region having one or more amino acid substitutions that improve ADCC, e.g., substitutions at positions 298, 333, and/or 334 of the Fc region (EU numbering of residues).

In certain embodiments, the antibody variant comprises an Fc region having one or more amino acid substitutions that increase FcRn binding, e.g., substitutions at positions 252, and/or 254, and/or 256 of the Fc region (EU numbering of residues). In certain embodiments, the antibody variant comprises an Fc region having amino acid substitutions at positions 252, 254 and 256. In one embodiment, the substitutions are M252Y, S254T and T256E in the Fc region derived from the human IgG1 Fc region.

In certain embodiments, the antibody variant comprises an Fc region having amino acid substitutions that decrease FcγR binding, e.g., substitutions at positions 234 and 235 of the Fc region (EU numbering of residues). In one embodiment, the substitutions are L234A and L235A (LALA). In certain embodiments, the antibody variant further comprises D265A and/or P329G in the Fc region derived from the human IgG1 Fc region. In one embodiment, the substitutions are L234A, L235A and P329G (LALA-PG) in the Fc region derived from the human IgG1 Fc region (see, e.g., WO 2012/130831 A1). In another embodiment, the substitutions are L234A, L235A and D265A (LALA-DA) in the Fc region derived from the human IgG1 Fc region.

In some embodiments, alterations are made to the Fc region that result in altered (i.e., improved or reduced) C1q binding and/or complement dependent cytotoxicity (CDC), e.g., as recorded in U. S. Patent No. 6,194,551, WO 99/51642, and Idusogie et al., J. Immunol. 164: 4178-4184 (2000).

Antibodies having increased half-lives and improved binding to the neonatal Fc receptor (FcRn) are recorded in US 2005/0014934 A1 (Hinton et al.), and the neonatal Fc receptor (FcRn) is responsible for the transfer of maternal IgG to the fetus (Guyer et al., J. Immunol. 117: 587 (1976) and Kim et al., J. Immunol. 24: 249 (1994)). Those antibodies comprise an Fc region having one or more substitutions therein that improve the binding of the Fc region to FcRn. Such Fc variants include those having substitutions at one or more of Fc region residues 238, 252, 254, 256, 265, 272, 286, 303, 305, 307, 311, 312, 317, 340, 356, 360, 362, 376, 378, 380, 382, 413, 424 or 434, e.g., substitution of Fc region residue 434 (see, e.g., U. S. Patent No. 7,371,826; Dall'Acqua, W. F., et al., J. Biol. Chem. 281 (2006) 23514-23524).

Fc region residues that are critical for mouse Fc-mouse FcRn interaction have been identified by site-directed mutagenesis (see, e.g., Dall'Acqua, W. F., et al., J. Immunol 169 (2002) 5171-5180). Residues I253, H310, H433, N434, and H435 (according to the EU numbering of Kabat) are involved in the interaction (Medesan, C. et al., Eur. J. Immunol. 26 (1996) 2533; Firan, M. et al., Int. Immunol. 13 (2001) 993; Kim, J. K. et al., Eur. J. Immunol. 24 (1994) 542). Residues I253, H310 and H435 are found to be critical for the interaction of human Fc with murine FcRn (Kim, J. K. et al., Eur. J. Immunol. 29 (1999) 2819). Studies of the human Fc-human FcRn complex showed that residues I253, S254, H435 and Y436 are critical for the interaction (Firan, M. et al., Int. Immunol. 13 (2001) 993; Shields, R.L. et al., J. Biol. Chem. 276 (2001) 6591-6604). A variety of mutants of residues 248 to 259 and 301 to 317 and 376 to 382 and 424 to 437 have been reported and examined (Yeung, Y. A. et al., J. Immunol. 182 (2009) 7667-7671).

In certain embodiments, the antibody variant comprises an Fc region having one or more amino acid substitutions that reduce FcRn binding, e.g., substitutions at positions 253, and/or 310, and/or 435 of the Fc region (EU numbering of residues). In certain embodiments, the antibody variant comprises an Fc region having amino acid substitutions at positions 253, 310 and 435. In one embodiment, the substitutions are I253A, H310A and H435A in the Fc region derived from the human IgG1 Fc region. See, e.g., Grevys, A. et al., J. Immunol. 194 (2015) 5497-5508.

In certain embodiments, the antibody variant comprises an Fc region having one or more amino acid substitutions that reduce FcRn binding, e.g., substitutions at positions 310, and/or 433, and/or 436 of the Fc region (EU numbering of residues). In certain embodiments, the antibody variant comprises an Fc region having amino acid substitutions at positions 310, 433 and 436. In one embodiment, the substitutions are H310A, H433A and Y436A in the Fc region derived from the human IgG1 Fc region (see, e.g., WO 2014/177460 A1).

See also Duncan and Winter, Nature 322: 738-40 (1988); U. S. Patent No. 5,648,260; U. S. Patent No. 5,624,821; and WO 94/29351, which are concerned with other examples of Fc region variants.

## B. Recombinant methods and compositions

Antibodies may be generated using recombinant methods and compositions, e.g., as recorded in US 4,816,567. For these methods, one or more isolated nucleic acids encoding an antibody are provided.

In the case of a natural antibody or a natural antibody fragment, two nucleic acids are required, one for a light chain or a fragment thereof and one for a heavy chain or a fragment thereof. Such nucleic acids encode an amino acid sequence comprising the VL and/or an amino acid sequence comprising the VH of an antibody (e.g., the light and/or heavy chains of the antibody). These nucleic acids can be on the same expression vector or on different expression vectors.

In the case of a bispecific antibody having a heterodimeric heavy chain, four nucleic acids are required, one for a first light chain, one for a second light chain comprising a first heteromonomeric Fc region polypeptide, one for a second light chain, and one for a second heavy chain comprising a second heteromonomeric Fc region polypeptide. The four nucleic acids can be comprised in one or more nucleic acid molecules or expression vectors. Such nucleic acids encode an amino acid sequence comprising a first VL of an antibody and/or an amino acid sequence comprising a first VH comprising a first heteromonomeric Fc region and/or an amino acid sequence comprising a second VL and/or an amino acid sequence comprising a second VH comprising a second heteromonomeric Fc region (e.g., the first and/or second light and/or first and/or second heavy chain of an antibody). These nucleic acids can be on the same expression vector or on different expression vectors. Normally, these nucleic acids are located on two or three expression vectors, i.e., one vector may comprise more than one of these nucleic acids. Examples of such bispecific antibodies are CrossMab and T cell bispecific agents (see, e.g., Schaefer, W. et al, PNAS, 108 (2011) 11187-1191). For example, one of the heteromonomeric heavy chains comprises a so-called "knob mutation" (T366W and optionally one of S354C or Y349C) and the other comprises a so-called "hole mutation" (T366S, L368A and Y407V and optionally Y349C or S354C) (see, e.g., Carter, P. et al., Immunotechnol. 2 (1996) 73).

In one embodiment, an isolated nucleic acid encoding an antibody used in the methods reported herein is provided.

In yet another embodiment, one or more vectors (e.g., expression vectors) comprising such nucleic acids are provided.

In yet another embodiment, a host cell comprising such nucleic acids is provided.

In one such embodiment, the host cell comprises (e.g., has been transformed with):

- In the case of an antibody composed of two identical light chains and two identical heavy chains that are disulfide-bonded comprising fragments of VH and VL:

   (1) a vector comprising a nucleic acid that encodes an amino acid sequence comprising the VL of the antibody and an amino acid sequence comprising the VH of the antibody, or
   (2) a first vector comprising a nucleic acid that encodes an amino acid sequence comprising the VL of the antibody and a second vector comprising a nucleic acid that encodes an amino acid sequence comprising the VH of the antibody.

- In the case of a bispecific antibody having heterodimeric heavy chains:

   (1) a first vector comprising a first pair of nucleic acids that encode amino acid sequences, one of the amino acid sequences comprising a first VL and the other comprising a first VH of an antibody, and a second vector comprising a second pair of nucleic acids that encode amino acid sequences, one of the amino acid sequences comprising a second VL and the other comprising a second VH of an antibody, or
   (2) a first vector comprising a first nucleic acid that encodes an amino acid sequence comprising one of the variable domains (preferably a light chain variable domain), a second vector comprising a pair of nucleic acids that encode amino acid sequences, one of the amino acid sequences comprising a light chain variable domain and the other comprising a first heavy chain variable domain, and a third vector comprising a pair of nucleic acids that encode amino acid sequences, one of the amino acid sequences comprising another light chain variable domain corresponding to the second vector and the other comprising a second heavy chain variable domain, or
   (3) a first vector comprising a nucleic acid that encodes an amino acid sequence comprising a first VL of an antibody, a second vector comprising a nucleic acid that encodes an amino acid sequence comprising a first VH of an antibody, a third vector comprising a nucleic acid that encodes an amino acid sequence comprising a second VL of an antibody, and a fourth vector comprising a nucleic acid that encodes an amino acid sequence comprising a second VH of an antibody.

In one embodiment, the host cell is eukaryotic, e.g., a Chinese hamster ovary (CHO) cell or a lymphoid cell (e.g., a Y0, NS0, Sp20 cell). In one embodiment, a method for generating an anti-RSV antibody is provided, wherein the method comprises culturing a host cell comprising a nucleic acid encoding the antibody, as provided above, under conditions suitable for expression of the antibody, and optionally recovering the antibody from the host cell (or host cell culture medium).

For the recombinant generation of an anti-RSV antibody, a nucleic acid encoding the antibody (e.g., as described above) is isolated and inserted into one or more vectors for further cloning and/or expression in a host cell. Such nucleic acids can be readily isolated and sequenced using conventional procedures (e.g., by using oligonucleotide probes capable of specifically binding to genes encoding the heavy and light chains of the antibody), or generated by recombinant methods or obtained by chemical synthesis.

Suitable host cells for cloning or expressing antibody-encoding vectors include prokaryotic or eukaryotic cells described herein. For example, antibodies can be generated in bacteria, in particular when glycosylation and Fc effector functions are not needed. For the expression of antibody fragments and polypeptides in bacteria, see, e.g., US 5,648,237, US 5,789,199 and US 5,840,523 (see also Charlton, K. A., in Methods in Molecular Biology, Vol. 248, Lo, B. K. C., ed. Humana Press, Totowa, NJ (2003), pp. 245-254, describing the expression of antibody fragments in *E. coli.).* After the expression, the antibody can be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized", resulting in the generation of antibodies with partially or fully human glycosylation patterns. See Gerngross, T.U., Nat. Biotech. 22 (2004) 1409-1414; Li et al., Nat. Biotech. 24 (2006) 210-215.

Suitable host cells for the expression of glycosylated antibodies are also derived from multicellular organisms (invertebrates and vertebrates). Examples of invertebrate cells include plant and insect cells. A number of baculoviral strains have been identified, which can be used in conjunction with insect cells, particularly for the transfection of *Spodoptera frugiperda* cells.

Plant cell cultures can also be used as hosts. See, e.g., US 5,959,177, US 6,040,498, US 6,420,548, US 7,125,978 and US 6,417,429 (describing PLANTIBODIES™ technology for generating antibodies in transgenic plants).

Vertebrate cells can also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells, as recorded, for example, in Graham, F. L. et al., J. Gen Virol. 36 (1977) 59-74); baby hamster kidney (BHK) cells; mouse sertoli cells (TM4 cells, as recorded, for example, in Mather, J. P., Biol. Reprod. 23 (1980) 243-252); monkey kidney cells (CV1); African green monkey kidney cells (VERO-76); human cervical carcinoma cells (HELA); canine kidney cells (MDCK); buffalo rat liver cells (BRL 3A); human lung cells (W138); human hepatocytes (Hep G2); mouse mammary tumor (MMT 060562); TRI cells, as recorded, e.g., in Mather, J. P. et al., Annals N. Y. Acad. Sci. 383 (1982) 44-68; MRC 5 cells; and FS4 cells. Other useful mammalian host cell lines include Chinese hamster ovary (CHO) cells, including DHFR⁻ CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody generation, see, e.g., Yazaki, P. and Wu, A.M., Methods in Molecular Biology, Vol. 248, Lo, B. K. C., ed., Humana Press, Totowa, NJ (2004), pages 255-268.

### C. Assays

The anti-RSV antibody provided herein can be identified, screened, or characterized for physical/chemical properties and/or biological activity by a variety of assays known in the art.

### 1. Binding assays and other assays

In one aspect, the antibody of the present invention is tested for its antigen-binding activity, for example, by known methods such as ELISA and Western blotting.

In another aspect, competition assays can be used to identify antibodies that compete with a reference anti-RSV antibody for binding to RSV or an antigen thereof. In certain embodiments, such competitive antibodies bind to the same epitope (e.g., a linear or conformational epitope) that is bound by a reference anti-RSV antibody. Detailed exemplary methods for mapping epitopes to which antibodies bind are recorded in Morris (1996) "Epitope Mapping Protocols", Methods in Molecular Biology vol. 66 (Humana Press, Totowa, NJ).

In an exemplary competition assay, immobilized RSV or an antigen thereof is incubated in a solution comprising a first labeled antibody (that binds to RSV or an antigen thereof) (e.g., a reference anti-RSV antibody) and a second unlabeled antibody (that is to be tested for its ability to compete with the first antibody for binding to the RSV or the antigen thereof). The second antibody can be present in a hybridoma supernatant. As a control, immobilized RSV or an antigen thereof is incubated in a solution comprising the first labeled antibody but not the second unlabeled antibody. After incubation under conditions permissive for binding of the first antibody to RSV or the antigen thereof, excess unbound antibody is removed, and the amount of label associated with the immobilized RSV or the antigen thereof is measured. If the amount of label associated with the immobilized RSV or the antigen thereof is substantially reduced in the test sample relative to the control sample, then this indicates that the second antibody is competing with the first antibody for binding to the RSV or the antigen thereof. See Harlow and Lane (1988) Antibodies: A Laboratory Manual ch.14 (Cold Spring Harbor Laboratory, Cold Spring Harbor, NY).

### 2. Activity assays

In one aspect, an assay for identifying anti-RSV antibodies having biological activity is provided. Biological activities can include, for example, the inhibition of RSV from infecting host cells by anti-RSV antibodies. Antibodies having such biological activities *in vivo* and/or *in vitro* are also provided.

### D. Methods and compositions for diagnosis and detection

In certain embodiments, any of the anti-RSV antibodies provided herein can be used to detect the presence of RSV in a biological sample. As used herein, the term "detection" encompasses quantitative or qualitative detection. In certain embodiments, the biological sample comprises a cell or tissue, such as a tumor tissue.

In one embodiment, an anti-RSV antibody for use in a diagnostic or detection method is provided. In yet another aspect, a method for detecting the presence of RSV in a biological sample is provided. In certain embodiments, the method comprises bringing a biological sample into contact with an anti-RSV antibody under conditions permissive for binding of the anti-RSV antibody to RSV or an antigen thereof, as described herein, and detecting whether a complex is formed between the anti-RSV antibody and the RSV or the antigen thereof. Such methods can be *in-vitro* or *in-vivo* methods. In one embodiment, an anti-RSV antibody is used to select a subject suitable for treatment with the anti-RSV antibody, e.g., wherein the patient is infected with RSV, or is suspected of being infected with RSV, or at risk of exposure to RSV.

In certain embodiments, a labeled anti-RSV antibody is provided. Labels include, but are not limited to, labels or moieties that are detected directly (such as fluorescent, chromogenic, electron-dense, chemiluminescent, and radioactive labels), and moieties, such as enzymes or ligands, that are detected indirectly, e.g., via enzymatic reactions or molecular interactions. Exemplary labels include, but are not limited to, radioisotopes $^{32}$P, $^{14}$C, $^{125}$I, $^{3}$H, and$^{131}$I, fluorophores such as rare earth chelates or fluorescein and derivatives thereof, rhodamine and derivatives thereof, dansyl, umbelliferone, luciferase (such as firefly luciferase and bacterial luciferase (U. S. Patent No. 4,737,456)), luciferin, 2,3-dihydrophthalazinedione, horseradish peroxidase (HRP), alkaline phosphatase, β-galactosidase, glucoamylase, lysozyme, saccharide oxidase (such as glucose oxidase, galactose oxidase, and glucose-6-phosphate dehydrogenase), heterocyclic oxidase (such as uricase and xanthine oxidase (coupled to an enzyme such as HRP that oxidize dye precursors using hydrogen peroxide)), lactoperoxidase, or microperoxidase, biotin/avidin, spin labels, phage labels, stable free radicals, etc.

## E. Pharmaceutical formulations

Pharmaceutical formulations of the anti-RSV antibody as described herein are prepared by mixing such antibody having the desired purity with one or more optional pharmaceutically acceptable carriers (Remington's Pharmaceutical Sciences, 16th edition, Osol, A. ed. (1980)), in the form of lyophilized formulations or aqueous solutions. Pharmaceutically acceptable carriers are generally nontoxic to recipients at the dosages and concentrations employed, and include, but are not limited to, buffers such as phosphate, citrate, and other organic acids; antioxidants, including ascorbic acid and methionine; preservatives (such as octadecyl dimethyl benzyl ammonium chloride; hexane diamine chloride; benzalkonium chloride, benzethonium chloride; phenols, butanol or benzyl alcohol; alkyl parabens such as methyl or propyl paraben; catechol; resorcinol; cyclohexanol; 3-pentanol; and m-cresol); low molecular weight (less than about 10 residues) polypeptides; proteins such as serum albumin, gelatin or immunoglobulin; hydrophilic polymers such as polyvinylpyrrolidone; amino acids such as glycine, glutamine, asparagine, histidine, arginine or lysine; monosaccharides, disaccharides and other carbohydrates, including glucose, mannose or dextrin; chelating agents such as EDTA; sugars such as sucrose, mannitol, trehalose or sorbitol; salt-forming counterions such as sodium; metal complexes such as Zn-protein complexes; and/or nonionic surfactants such as polyethylene glycol (PEG). Exemplary pharmaceutically acceptable carriers herein further include interstitial drug dispersants such as soluble neutral active hyaluronidase glycoprotein (sHASEGP), e.g., human soluble PH-20 hyaluronidase glycoprotein, such as rHuPH20 (HYLENEX®, Baxter International, Inc.). Certain exemplary sHASEGP and methods of use, including rHuPH20, are recorded in U. S. Patent Publication Nos. 2005/0260186 and 2006/0104968. In one aspect, sHASEGP is combined with one or more additional glycosaminoglycanases such as chondroitinase.

Exemplary lyophilized antibody formulations are recorded in U. S. Patent No. 6,267,958. Aqueous antibody formulations include those recorded in U. S. Patent No. 6,171,586 and WO 2006/044908, the latter formulation comprising a histidine-acetate buffer.

The formulations herein may also contain more than one active ingredients necessary for the particular indication being treated, preferably those with complementary activities that do not adversely affect each other. Such active ingredients are suitably present in combination in amounts that are effective for the purpose intended.

The active ingredient may be encapsulated in microcapsules prepared, for example, by coacervation techniques or by interfacial polymerization (e.g., hydroxymethylcellulose or gelatin microcapsules and poly(methyl methacrylate) microcapsules, respectively), in colloidal drug delivery systems (e.g., liposomes, albumin microspheres, microemulsions, nanoparticles and nanocapsules), or in macroemulsions. Such techniques are disclosed in Remington's Pharmaceutical Sciences, 16th edition, Osol, A. ed. (1980).

Sustained-release preparations can be prepared. Suitable examples of sustained-release preparations include semipermeable matrices of solid hydrophobic polymers containing an antibody, which matrices are in the form of shaped articles, e.g. films, or microcapsules.

Formulations to be used for *in-vivo* administration are generally sterile. Sterility can be readily accomplished, e.g., by filtration through sterile filtration membranes.

## F. Preventive, therapeutic methods and compositions

Any of the anti-RSV antibodies provided herein can be used in preventive or therapeutic methods.

In one aspect, an anti-RSV antibody for use as a medicament is provided. In yet other aspects, anti-RSV antibodies for use in the prevention or treatment of RSV infection are provided. In certain embodiments, anti-RSV antibodies for use in preventive or therapeutic methods are provided. In certain embodiments, the present invention provides an anti-RSV antibody for use in a method for the prevention of an individual at risk of an RSV infection, the prevention comprising administering to the individual an effective amount of the anti-RSV antibody. In certain embodiments, the present invention provides an anti-RSV antibody for use in a method for the treatment of an individual having an RSV infection, the treatment comprising administering to the individual an effective amount of the anti-RSV antibody. In one embodiment, the antibody is for use in treating an RSV infection or delaying its development.

An "individual" according to any of the above embodiments is preferably a human. In yet another aspect, the present invention provides the use of an anti-RSV antibody in the manufacture or preparation of a medicament. In one embodiment, the medicament is for use in treatment of an RSV infection. In yet another embodiment, the medicament is for use in a method for treating an RSV infection, the method comprising administering to an individual having the RSV infection an effective amount of the medicament. An "individual" according to any of the above embodiments may be a human.

As used herein, the term "RSV infection" may be, for example, a lower respiratory tract infection.

In yet another aspect, the present invention provides a method for treating an RSV infection. In one embodiment, the method comprises administering to an individual having a cancer an effective amount of anti-RSV. An "individual" according to any of the above embodiments may be a human.

In yet another aspect, the present invention provides a pharmaceutical formulation comprising any of the anti-RSV antibodies provided herein, e.g., for use in any of the above therapeutic methods. In one embodiment, the pharmaceutical formulation comprises any of the anti-RSV antibodies provided herein and a pharmaceutically acceptable carrier.

In yet another aspect, the present invention provides a pharmaceutical formulation comprising any of the anti-RSV antibodies provided herein, e.g., for use in any of the above therapeutic methods. In one embodiment, the pharmaceutical formulation comprises any of the anti-RSV antibodies provided herein and a pharmaceutically acceptable carrier. In another embodiment, the pharmaceutical formulation comprises any of the anti-RSV antibodies provided herein and at least one additional therapeutic agent. In one embodiment, the at least one additional therapeutic agent is, for example, another anti-RSV antibody that binds to a different epitope of RSV than the anti-RSV antibody herein.

The antibody of the present invention may be used alone or in combination with other agents in a therapy. For example, the antibody of the present invention may be co-administered with at least one additional therapeutic agent.

Such combination therapies noted above encompass combined administration (where two or more therapeutic agents are contained in the same or different formulations), and separate administration, in which case the administration of the antibody of the present invention may occur prior to, simultaneously with, and/or after the administration of one or more additional therapeutic agents. In one embodiment, the administration of the anti-RSV antibody and the administration of an additional therapeutic agent occur within about one month, or within about one, two or three weeks, or within about one, two, three, four, five or six days, of each other.

The antibody of the present invention (and any additional therapeutic agent) can be administrated by any suitable means, including parenteral, intrapulmonary, and intranasal, and, if desired for topical treatment, intralesional administration. Parenteral infusion includes intramuscular, intravenous, intraarterial, intraperitoneal, or subcutaneous administration. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are encompassed herein.

The antibody of the present invention would be formulated, dosed and administered in a manner consistent with good medical practice. Factors for consideration in this context include the particular disorder being treated, the particular mammal being treated, the clinical condition of the individual patient, the cause of the disorder, the site of drug delivery, the method of administration, the scheduling of administration, and other factors known to medical practitioners. The antibody need not be, but may optionally be formulated with one or more agents currently used to prevent or treat the disorder. The effective amount of such other agents described above depends on the amount of the antibody present in the formulation, the type of disorder or treatment, and other factors discussed above. These agents are generally used in the same dosage and route of administration as described herein, or in about 1%-99% of the dosage described herein, or in any dosage and by any route, the dosage and route being empirically/clinically determined to be appropriate.

For the prevention or treatment of a disease, an appropriate dosage of the antibody of the present invention (when used alone or in combination with one or more other additional therapeutic agents) will depend on the type of a disease to be treated, the type of the antibody, the severity and course of the disease, whether the antibody is administered for preventive or therapeutic purposes, previous treatment, the patient's clinical history and response to the antibody, and the discretion of the attending physician. The antibody is suitably administered to the patient at one time or over a series of treatments. Depending on the type and severity of the disease, about 1 μg/kg-15 mg/kg (e.g., 0.1 mg/kg-10 mg/kg) of the antibody can be an initial candidate dosage for administration to the patient, whether, for example, by one or more separate administrations, or by continuous infusion. One typical daily dosage may range from about 1 μg/kg to 100 mg/kg or more, depending on the factors mentioned above. For repeated administrations over several days or longer, depending on the condition, the treatment would generally be sustained until the desired suppression of disease symptoms occurs. One exemplary dosage of an antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to a patient. The above doses may be administered intermittently, e.g., once a week or once every three weeks (e.g., such that the patient receives about 2 doses to about 20 doses, or for example, about 6 doses of the antibody). An initial higher loading dose, followed by one or more lower doses may be administered. An exemplary dosing regimen includes administration. However, other dosage regimens may be useful. The progress of this treatment is easily monitored by conventional techniques and assays.

It should be understood that any of the above formulations or therapeutic methods may be practiced using the immunoconjugate of the present invention in place of or in addition to the anti-RSV antibody.

## G. Preparation

In another aspect of the present invention, a preparation comprising material useful for the treatment, prevention and/or diagnosis of the disorder described above. The preparation comprises a container and a label or package insert on or associated with the container. Suitable containers include, for example, bottles, vials, syringes, IV solution bags, etc. The containers can be formed from a variety of materials such as glass or plastic. The container holds a composition which is by itself or combined with another composition effective for treating, preventing and/or diagnosing the condition and may have a sterile access port (for example, the container may be a vial or an intravenous solution bag having a stopper pierceable by a hypodermic injection needle). At least one active agent in the composition is the antibody of the present invention. The label or package insert indicates that the composition is used for treating the condition of choice. Moreover, the preparation may comprise: (a) a first container with a composition contained therein, wherein the composition comprises the antibody of the present invention; and (b) a second container with a composition contained therein, wherein the composition comprises a further therapeutic agent. The preparation in this embodiment of the present invention may further comprise a package insert indicating that the composition may be used to treat a particular condition. Alternatively/additionally, the preparation may further comprise a second (or third) container comprising a pharmaceutically acceptable buffer, such as bacteriostatic water for injection (BWFI), phosphate buffered saline, Ringer's solution and dextrose solution. The preparation may further comprise other materials desirable from a commercial and user standpoint, including other buffers, diluents, filters, needles, and syringes.

## Description of amino acid sequences and nucleic acid sequences

| No. | | | Description |
|---|---|---|---|
| 1 | F6-10 | CDR-H1 | Amino acid sequence |
| 2 | F6-10 | CDR-H2 | Amino acid sequence |
| 3 | F6-10 | CDR-H3 | Amino acid sequence |
| 4 | F6-10 | CDR-L1 | Amino acid sequence |
| 5 | F6-10 | CDR-L2 | Amino acid sequence |
| 6 | F6-10 | CDR-L3 | Amino acid sequence |
| 7 | F6-10 | VH | Amino acid sequence |
| 8 | F6-10 | VL | Amino acid sequence |
| 9 | F6-18 | CDR-H1 | Amino acid sequence |
| 10 | F6-18 | CDR-H2 | Amino acid sequence |
| 11 | F6-18 | CDR-H3 | Amino acid sequence |
| 12 | F6-18 | CDR-L1 | Amino acid sequence |
| 13 | F6-18 | CDR-L2 | Amino acid sequence |
| 14 | F6-18 | CDR-L3 | Amino acid sequence |
| 15 | F6-18 | VH | Amino acid sequence |

(continued)

| No. | | | Description |
|---|---|---|---|
| 16 | F6-18 | VL | Amino acid sequence |
| 17 | F6-113 | CDR-H1 | Amino acid sequence |
| 18 | F6-113 | CDR-H2 | Amino acid sequence |
| 19 | F6-113 | CDR-H3 | Amino acid sequence |
| 20 | F6-113 | CDR-L1 | Amino acid sequence |
| 21 | F6-113 | CDR-L2 | Amino acid sequence |
| 22 | F6-113 | CDR-L3 | Amino acid sequence |
| 23 | F6-113 | VH | Amino acid sequence |
| 24 | F6-113 | VL | Amino acid sequence |
| 25 | F6-125 | CDR-H1 | Amino acid sequence |
| 26 | F6-125 | CDR-H2 | Amino acid sequence |
| 27 | F6-125 | CDR-H3 | Amino acid sequence |
| 28 | F6-125 | CDR-L1 | Amino acid sequence |
| 29 | F6-125 | CDR-L2 | Amino acid sequence |
| 30 | F6-125 | CDR-L3 | Amino acid sequence |
| 31 | F6-125 | VH | Amino acid sequence |
| 32 | F6-125 | VL | Amino acid sequence |
| 33 | F6-10 F6-18 F6-113 | CL | Amino acid sequence |
| 34 | F6-125 | CL | Amino acid sequence |
| 35 | IgG1 | CH | Amino acid sequence |
| 36 | F6-10 | VH | Nucleotide sequence |
| 37 | F6-10 | VL | Nucleotide sequence |
| 38 | F6-18 | VH | Nucleotide sequence |
| 39 | F6-18 | VL | Nucleotide sequence |
| 40 | F6-113 | VH | Nucleotide sequence |
| 41 | F6-113 | VL | Nucleotide sequence |
| 42 | F6-125 | VH | Nucleotide sequence |
| 43 | F6-125 | VL | Nucleotide sequence |
| 44 | RSV A2 pre-F | | Amino acid sequence |

The present invention provides a set of antibodies having an affinity for RSV A2 pre-F protein between 0.001 nM and 3.25 nM, and a neutralizing activity IC50 for RSV A2 strain between 0.34 ng/mL and 80.04 ng/mL, a neutralizing activity IC50 for RSV B9320 strain between 3.65 ng/mL and 153.90 ng/mL, and a neutralizing activity IC50 for RSV B18537 strain between 1.87 ng/mL and 67.03 ng/mL.

**Example 1: Preparation of anti-RSV antibodies**

Peripheral blood was collected from healthy adult volunteers, and upper plasma and middle PBMC were obtained by density gradient centrifugation. Memory B cells that specifically bind to pre-F protein were isolated from PBMC by flow sorting with fluorescently labeled pre-F protein, and transfectable PCR fragments with expression activity were obtained by nested PCR. These fragments were transfected into CHO cells for expression, and cell supernatants containing secreted antibodies were obtained. Hundreds of pre-F binding positive clones were obtained by screening for binding activity by ELISA. Multiple recombinant antibodies were tested for neutralizing activity against the RSV A2 strain. Based on the neutralizing activities against RSV A2, RSV 9320 and RSV 18537, and affinities for RSV A2 pre-F protein of each recombinant antibody, four antibodies, namely F6-10, F6-18, F6-113 and F6-125, were finally selected.
The amino acid sequences of CDRs of the antibodies are as shown in Table 1.

Table 1: Amino acid sequences of CDRs of anti-RSV antibodies

|  |  | CDR1 | CDR2 | CDR3 |
|---|---|---|---|---|
| F6-10 | HCDR | EFTFNIHA | ISSTGEGT | ARYSFRYGELRGTFDI |
|  | LCDR | QSVGDS | DTT | QQYDESWT |
| F6-18 | HCDR | GFSVSDYG | IRSHGDGT | ARDRVWAHDYDDTAWLDAFDL |
|  | LCDR | QSVGSL | DAS | QQYKSHSPP |
| F6-113 | HCDR | GASVSDDD | IDQTGSF | ARGGLVVTDVGQGWDAFDV |
|  | LCDR | QDIDNW | AAS | QQGDSFPLT |
| F6-125 | HCDR | GYTFSDNA | ITANNGNT | AREVVNYGLGSLHFDY |
|  | LCDR | KLGNDY | EDA | QAWDGTTVI |

The amino acid sequences of VH, VL and CL of the antibodies are as shown in Table 2.

Table 2: Amino acid sequences of VH, VL and CL of anti-RSV antibodies

|  | VH | VL | CL |
|---|---|---|---|
| F6-10 | DALLLQSGGGLMQPGGS LRLSCAASEFTFNIHAMT WVRQSPGKGLEWVSGISS TGEGTYYTDSVKGRFTIS RDNSKSTLYLQMNSLRAE DTATYFCARYSFRYGELR GTFDIWGQGVLVSVSS | DIQMTQTPSTLSASVGDR VIITCRASQSVGDSVAWF QQKPGRAPKLLIYDTTNL ERGVPSRFSGSFSGTAFTL TITRLQPDDFATYYCQQY DESWTFGQGTKVEVK | RTVAAPSVFIFPPSDEQLK SGTASVVCLLNNFYPREA KVQWKVDNALQSGNSQE SVTEQDSKDSTYSLSSTLT LSKADYEKHKLYACEVT HQGLSSPVTKSFNRGEC |
| F6-18 | EEQLVESGGGLVRPGGSL RLSCVASGFSVSDYGMH WVRQAPGRGLEYVSAIRS HGDGTYYVNSVKGRFIIS RDNSKNTLYLQMGSLSAE DMAVYYCARDRVWAHD YDDTAWLDAFDLWGQGT TVIVSS | DIQMTQSPSTLSASVGDK VTITCRASQSVGSLLAWY QQKPGKAPKLLIYDASNL QRGVPSRFSGSGSGTEFTL TISSLQPDDFATYYCQQY KSHSPPFGQGTKLEIK | RTVAAPSVFIFPPSDEQLK SGTASVVCLLNNFYPREA KVQWKVDNALQSGNSQE SVTEQDSKDSTYSLSSTLT LSKADYEKHKLYACEVT HQGLSSPVTKSFNRGEC |
| F6-113 | QVQLQQWGAGLLKPSET LSLTCAVDGASVSDDDWT WIRQSPGKGLEWIGQIDQ TGSFNYNPSLRSRVTISPD TSKNHFSLSLTSVTAADTA LYYCARGGLVVTDVGQG WDAFDVWGQGTMVTVS S | DIQLTQSPSSVSASVGDRI TITCRASQDIDNWLAWYQ QKPGEAPKLLIYAASTLQS GVPSRFSGSGAGTEFTLTI TSLQPEDFAGYFCQQGDS FPLTFGGGTKVDIK | RTVAAPSVFIFPPSDEQLK SGTASVVCLLNNFYPREA KVQWKVDNALQSGNSQE SVTEQDSKDSTYSLSSTLT LSKADYEKHKLYACEVT HQGLSSPVTKSFNRGEC |

(continued)

|  | VH | VL | CL |
|---|---|---|---|
| F6-125 | QLQLVQSGTEVKKPGASV KVSCKASGYTFSDNAINW VRQAPGQGLEWLGWITA NNGNTNYAQKIQGRVTM TTDTSTSTVYMELRSLGS DDTAVYYCAREVVNYGL GSLHFDYWGQGTLVTVS S | SYELTQPPSVSVSPGQTVS ISCSGDKLGNDYASWYQ QKPGQSPLLVIYEDAKRP SGIPERFSGSNSGNTATLTI SGTQAMDDADYYCQAW DGTTVIFGGGTKLTVL | GQPKAAPSVTLFPPSSEEL QANKATLVCLISDFYPGA VTVAWKADSSPVKAGVE TTTPSKQSNNKYAASSYL SLTPEQWKSHRSYSCQVT HEGSTVEKTVAPTECS |

The coding sequences of the light and heavy chain variable regions of the antibodies were cloned into eukaryotic expression vectors carrying the coding sequence of the human IgG1 constant region. The resulting vector was transiently transfected into CHO cells for secretory expression. Antibody proteins with a purity of > 90%, namely F6-10, F6-18, F6-113 and F6-125, were obtained by protein A affinity purification.

Amino acid sequence of human IgG1 constant region:

ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGG
PSVFLFPPKPKDTLYITREPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKS
RWQQGNVFSCSVMHEALHNHYTQKSLSLSPGK*

Amino acid sequence of RSV A2 pre-F:

MELLILKANAITTILTAVTFCFASGQNITEEFYQSTCSAVSKGYLSALRTGWYTSVITIELSN
IKENKCNGTDAKVKLIKQELDKYKNAVTELQLLMQSTPPTNNRARRELPRFMNYTLNN
AKKTNVTLSKKRKRRFLGFLLGVGSAIASGVAVSKVLHLEGEVNKIKSALLSTNKAVVSL
SNGVSVLTFKVLDLKNYIDKQLLPILNKQSCSISNIETVIEFQQKNNRLLEITREFSVNAGV
TTPVSTYMLTNSELLSLINDMPITNDQKKLMSNNVQIVRQQSYSIMSIIKEEVLAYVVQLP
LYGVIDTPCWKLHTSPLCTTNTKEGSNICLTRTDRGWYCDNAGSVSFFPQAETCKVQSN
RVFCDTMNSLTLPSEINLCNVDIFNPKYDCKIMTSKTDVCSSVITSLGAIVSCYGKCKCTA
SNKNRGIIKTFSNGCDYVSNKGMDTVSVGNTLYYVNKQEGKSLYVKGEPIINFYDPLVFP
SDEFDASISQVNEKINQSLAFIRKSDELLHNVAGKSTTNIMITTIIIVIIVILLSLIAVGLLLY
CKARSTPVTLSKDQLSGINNIAFSN

**Example 2:** Characterization of anti-RSV antibodies - characterization of antigen-binding activity by ELISA

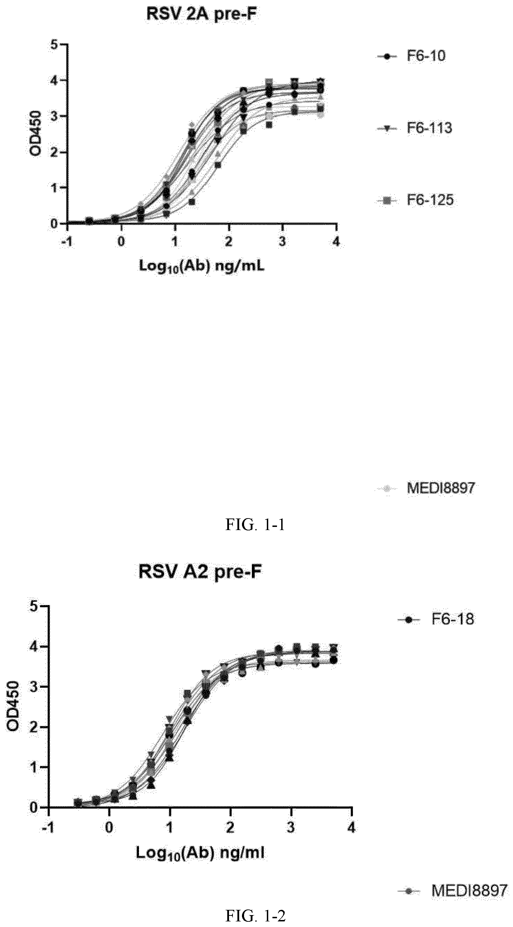

An RSV A2 pre-F protein (house-made by Vazyme) diluted to 2 μg/mL in a carbonate buffer pH 9.6 was added to a 96-well microplate (NEST, 504201) at 100 μL/well and coated at 4°C overnight. The solution was removed, and the plate was washed twice with PBST and blocked with a blocking solution (PBS + 5% BSA) at 37°C for 2 hours. The solution was removed, and 3-fold serial dilutions of the antibody (starting concentration: 5 μg/mL, a total of 10 gradients) in a diluent (PBS + 5% BSA) were added to the microwell plate at 100 μL/well and incubated at 37°C for 1 hour. The solution was removed, and the plate was washed 3 times with PBST. 100 μL of mouse anti-human IgG Fc-HRP (house-made by Vazyme) at 1 : 10000 dilution was added to each well and incubated at 37°C for 1 hour. The solution was removed, and the plate was washed 3 times with PBST. 100 μL of chromogenic substrate TMB was added to each well and incubated at 37°C for 10 minutes in the dark. The solution was removed, and the plate was washed 3 times with PBST. 50 μL of 2 M sulfuric acid was added to each well. The OD values at 450 nm were measured on a multifunctional microplate reader (Tecan, Spark). A four-parameter fitting was performed to calculate EC50 values (ng/mL) for the antigen-binding activity of the antibodies. The results are as shown in Table 3.1 and Table 3.2 (Table 3.2 and Table 3.1 are the results of different experiments, and the only difference in the operations of the experiment in Table 3.2 is "2-fold serial dilutions of the antibody (starting concentration: 1 μg/mL, a total of 14 gradients) in a diluent (PBS + 5% BSA)"). The antibody concentrations and OD values were plotted using GraphPad Prism 8.0. The results are as shown in FIG. 1-1 and FIG. 1-2.

Table 3.1: EC50 values (ng/mL) for RSV A2 pre-F binding activity of anti-RSV antibodies

| Antibody | F6-10 | F6-113 | F6-125 | MEDI8897 |
|----------|-------|--------|--------|----------|
| EC50 | 30.67 | 13.3 | 14.42 | 31.3 |

Table 3.2: EC50 value (ng/mL) for RSV A2 pre-F binding activity of antibody F6-18

| Antibody | F6-18 | MEDI8897 |
|----------|-------|----------|
| EC50 | 15.98 | 14.62 |

**Example 3: Characterization of anti-RSV antibodies - characterization of affinity by BLI**

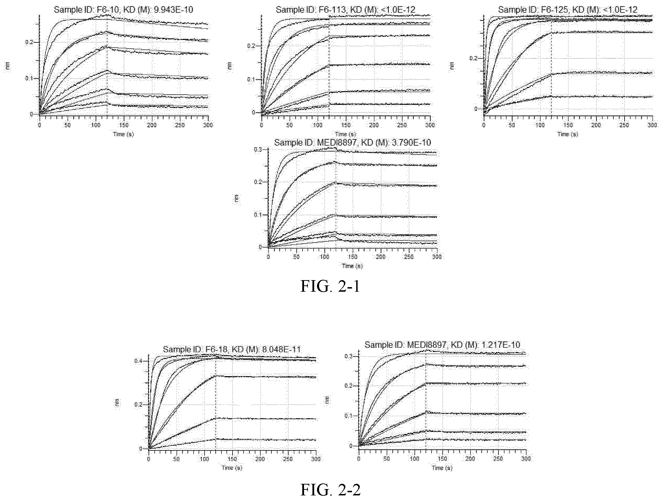

The affinity fitting curves for antibodies were obtained by bio-layer interferometry (BLI) using the Octet protein analysis system (SARTORIUS, Octet R8). The sensor used was HIS1K (SARTORIUS, 18-5120), the capture agent was 47.6 nM RSV A2 pre-F protein, the fixation time was 120 seconds, the antibody concentrations were 500 nM, 167 nM, 56 nM, 19 nM, 6 nM, 2 nM, and 0.69 nM, the association time was 60 seconds, the dissociation time was 120 seconds, the regeneration solution was 10 mM glycine-hydrochloric acid pH 1.5, and the regeneration time was 180 seconds. The KD values (M) for the antigen-binding affinity of the antibodies were calculated using the software Octet Analysis Studio 12.2. The results are as shown in Table 4.1 and Table 4.2. The software Octet BLI Discovery 12.2 was used for recording. The results are as shown in FIG. 2-1 and FIG. 2-2.

Table 4.1: KD values (M) for RSV A2 pre-F binding affinity of anti-RSV antibodies

| Antibody | F6-10 | F6-113 | F6-125 | MEDI8897 |
|----------|-------|--------|--------|----------|
| Affinity | 9.94 E-10 | <1.0 E-12 | <1.0 E-12 | 3.790 E-10 |

Table 4.2: KD value (M) for RSV A2 pre-F binding affinity of antibody F6-18

| Antibody | F6-18 | MEDI8897 |
|----------|-------|----------|
| Affinity | 8.05 E-11 | 1.22 E-10 |

**Example 4: Characterization of anti-RSV antibodies - characterization of neutralizing activity by *in-vitro* microneutralization assay**

3-fold serial dilutions of antibodies (starting concentration: 0.5 μg/mL, a total of 7 gradients) in PBS + 5% HIFBS were added to 96-well plates (NEST, 701001) containing viruses (RSV A2, ATCC VR-1540; RSV B9320, ATCC VR-955; RSV B18537, ATCC VR-1580) (60 μL/well, 500 pfu) at 60 μL/well, and incubated in a cell culture incubator (37°C, 5% $CO_2$)

for 1 hour. 50 μL/well of the above mixture was coated in 96-well plates (NEST, 701001) containing Hep2 cells with a confluence of 90% (coated at $3 \times 10^5$ cells/mL the day before), and incubated in a cell incubator (at 37°C, 5% $CO_2$) for 2 hours. The supernatant was removed. 100 μL of DMEM medium supplemented with 2% FB S and 1% penicillin-streptomycin (Solaibio, P1400) was added to each well, and the incubation continued for 21-22 hours. The supernatant was removed. 100 μL of 4% paraformaldehyde was added to each well, and the cells were fixed for 10 minutes. The paraformaldehyde was removed. The cells were washed once by adding 250 μL of PBS to each well, 100 μL of PBS + 4% BSA was added to each well, and blocking was performed in a cell culture incubator (37°C, 5% $CO_2$) for 30 minutes. The blocking solution was removed. 50 μL of 3D3 fluorescent antibody (house-made by Vazyme) diluted to 5 μg/mL was added to each well, and incubated in a cell culture incubator (37°C, 5% $CO_2$) for 1 hour. The antibody was removed. The plate was washed 3 times with PBST, and spin-dried. The number of spots per well was read using a fluorescence (enzyme-linked) immunospot analyzer (CTL, S6 Ultra M2). The wells containing only cells (cell wells) were used as negative wells, and the wells containing only viruses (virus wells) were used as positive wells.

$$\text{Virus neutralization of antibody [\%]} = \frac{\text{number of spots in virus wells} - \text{number of spots in antibody wells}}{\text{number of spots in virus wells} - \text{number of spots in cell wells}} \times 100$$

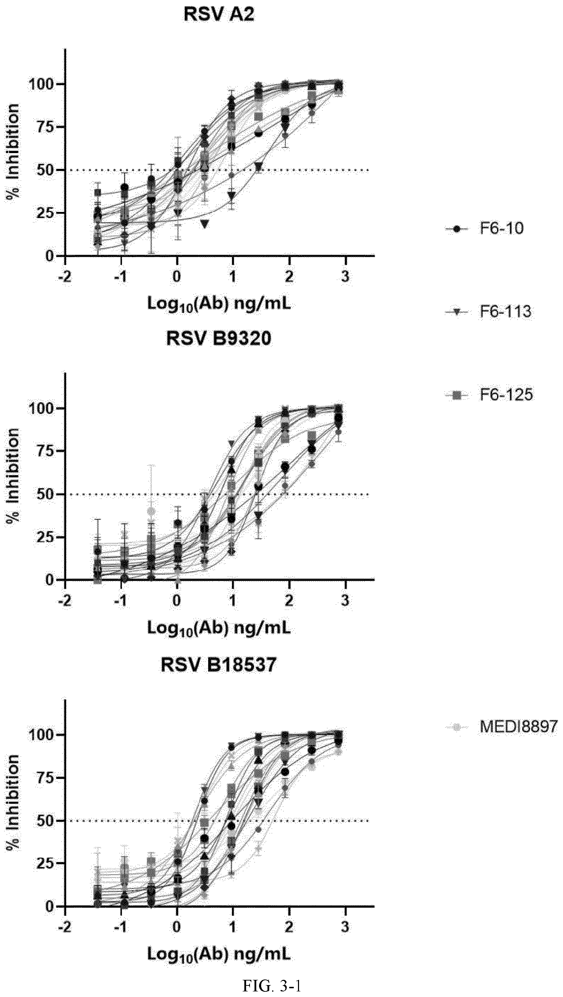

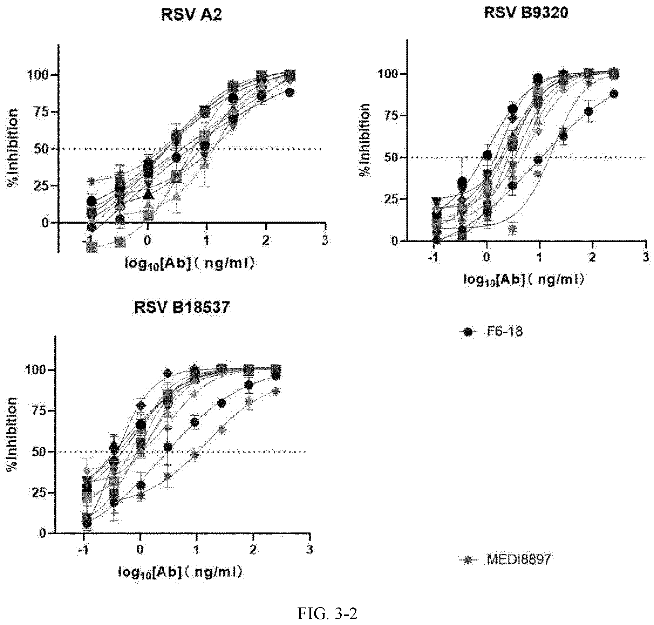

The antibody concentrations and virus neutralization were plotted using GraphPad Prism 8.0. The results are as shown in FIG. 3-1 and FIG. 3-2. A four-parameter fitting was performed to calculate IC50 values (ng/mL) for the virus neutralizing activity of the antibodies. The results are as shown in Table 5.1 and Table 5.2.

Table 5.1: IC50 values (ng/mL) for virus neutralizing activity of anti-RSV antibodies

|  | F6-10 | F6-113 | F6-125 | MEDI8897 |
|---|---|---|---|---|
| A2 | 1.39 | 2.41 | 2.94 | 4.67 |
| B9320 | 5.20 | 3.65 | 10.19 | 19.98 |
| B18537 | 2.42 | 1.87 | 4.54 | 34.46 |

Table 5.2: IC50 values (ng/mL) for virus neutralizing activity of antibody F6-18

|  | F6-18 | MEDI8897 |
|---|---|---|
| A2 | 0.34 | 4.13 |
| B9320 | 9.08 | 17.45 |
| B18537 | 2.45 | 15.20 |

**Example 5: Characterization of anti-RSV antibodies - characterization of epitopes by competitive binding**

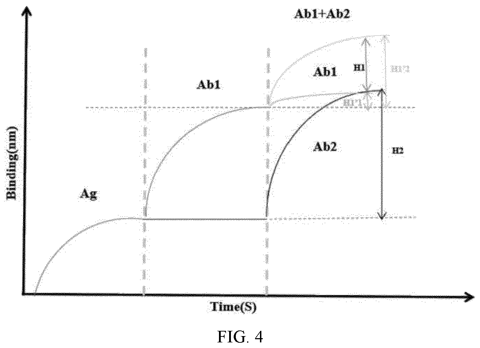

An RSV A2 pre-F protein was diluted to 10 μg/mL with a buffer (PBS + 0.02% Tween 20 + 0.1% BSA). The antibodies were diluted to 200 nM with the above buffer, both added at 200 μL/well. As shown in FIG. 4, the above buffer was run on an Octet protein analysis system (SARTORIUS, Octet R8) for 120 seconds to reach the baseline level, the antigen was run until the signal reached 0.3 nm, the above buffer was run for 60 seconds to reach the baseline level, the first antibody was run for 180 seconds, and the second antibody was run for 180 seconds (the same concentration of the first antibody was added to the second antibody to prevent false positive results due to the dissociation of the first antibody upon binding of the second antibody in the case where the first antibody was a fast-dissociating antibody). The regeneration solution (10 mM glycine-hydrochloric acid, pH 1.5) and the buffer were alternately run for a total of 20 seconds, repeating 3 times. The above cycle was repeated.

Data analysis:

the second signal was set to H1'2 when the first antibody was Ab1 and the second antibody was Ab2+Ab1;
the second signal was set to H1'1 when the first antibody was Ab1 and the second antibody was Ab1;
then
the second signal was H1, = H1'2 - H1'1 when the first antibody was Ab1 and the second antibody was Ab2;
the second signal was set to H2 when the first antibody was 0 and the second antibody was Ab2;
then

the inhibition on the second antibody by the first antibody was: 1 - (H1/H2) $\times$ 100%
(two antibodies were swapped to calculate the inhibition on the first antibody by the second antibody).

Experimental validity judgment:

self-reactive signal < 20%; otherwise, the data is invalid.

Decision criteria:

> 70%: complete competition, one direction meeting this criterion is sufficient;
30%-70%: partial competition;
< 30%: no competition at all, one direction meeting this criterion is sufficient.

When the test antibody completely competes with a reference antibody for a known epitope, the test antibody is considered to bind to the same epitope as the reference antibody.

The inhibition was calculated as described above. The results are as shown in Table 6.1 and Table 6.2. It is known that the reference antibody MEDI8897 binds to epitope Ø of the RSV 2A pre-F protein, Motavizumab binds to epitope II of the RSV 2A F protein, MPE8 binds to epitope III of the RSV 2A F protein, and MK-1654 binds to epitope IV of the RSV 2A F protein. The results show that F6-10 and F6-113 compete with MEDI8897, that is, these antibodies also recognize the epitope Ø of the F protein; F6-125 competes with MK-1654, that is, the antibody also recognizes the epitope IV of the pre-F protein; F6-18 competes with MK-1654, that is, the antibody also recognizes the epitope IV of the pre-F protein.

Table 6.1: Competitive binding of anti-RSV antibodies

|  | MEDI8897 | F6-10 | F6-113 |  |
|---|---|---|---|---|
| MEDI8897 | 96% | 90% | 94% |  |
| F6-10 | 95% | 95% |  |  |
| F6-113 | 99% |  | 95% |  |
|  | MK-1654 | F6-125 | Motavizumab | MPE8 |
| MK-1654 | 98% | 97% |  |  |
| F6-125 | 93% | 98% |  |  |
| Motavizumab |  |  | 97% | 98% |
| MPE8 |  |  | 85% | 97% |

Table 6.2: Competitive binding of antibody F6-18

|  | MEDI8897 | MK-1654 | F6-18 |
|---|---|---|---|
| MEDI8897 | 96% |  |  |
| MK-1654 |  | 100% | 91% |
| F6-18 |  | 98% | 98% |

Coding sequences of antibody variable regions

F6-10 VH GATGCGCTCCTGTTGCAGTCTGGGGGAGGCCTGATGCAGCCGG GGGGGTCCCTGAGACTCTCCTGTGCAGCCTCTGAATTCACTTTC AACATCCACGCCATGACGTGGGTCCGCCAGTCTCCTGGGAAGG GACTGGAGTGGGTCTCAGGTATCAGTAGCACGGGTGAAGGCAC CTATTACACAGACTCCGTGAAGGGCCGGTTCACCATCTCCAGAG ACAATTCCAAGAGTACTCTCTATCTTCAAATGAACAGCCTGCGG GCCGAGGACACGGCCACATATTTCTGTGCGAGATATTCCTTTCG GTATGGGGAACTACGGGGAACATTTGACATCTGGGGCCAGGGA GTCCTGGTCTCCGTCTCCTCA

VL GACATCCAGATGACCCAGACTCCCTCCACTCTGTCTGCGTCTGT TGGAGACAGAGTCATCATCACTTGCCGGGCCAGTCAGAGTGTT GGTGACTCGGTGGCCTGGTTTCAGCAGAAACCAGGGAGAGCCC CTAAACTCCTGATCTATGACACCACCAATTTAGAACGTGGGGTC CCATCAAGGTTCAGCGGCAGTTTCTCTGGGACAGCATTCACTCT CACCATCACCAGGTTGCAGCCTGATGATTTTGCAACTTATTACTG CCAACAATATGATGAATCTTGGACGTTCGGCCAAGGGACCAAG GTGGAGGTCAAA

F6-18 VH GAGGAGCAGCTGGTGGAGTCTGGGGGAGGCTTGGTCCGGCCG GGTGGGTCTCTGAGACTCTCCTGTGTAGCCTCTGGATTCAGTGT CAGTGACTATGGCATGCATTGGGTCCGCCAGGCTCCAGGGAGG GGACTGGAATATGTTTCGGCCATTAGAAGTCATGGGGATGGCAC ATATTATGTAAACTCTGTGAAGGGCAGGTTCATTATCTCCAGAG ACAATTCCAAGAATACGTTGTATCTTCAAATGGGCAGCCTGAGC GCTGAGGACATGGCTGTCTACTACTGTGCGAGAGATAGAGTCTG GGCCCACGACTACGATGACACCGCGTGGCTTGATGCTTTTGATC TCTGGGGCCAAGGGACAACGGTCATCGTCTCTTCA

(continued)

VL

GACATCCAGATGACCCAGTCTCCTTCCACCCTGTCTGCATCTGT
TGGAGACAAAGTCACCATCACCTGCCGGGCCAGTCAGAGTGTT
GGTAGCTTATTGGCCTGGTATCAGCAGAAACCAGGGAAAGCCC
CTAAACTCCTGATCTATGACGCCTCCAATTTGCAACGTGGGGTC
CCATCAAGGTTCAGCGGCAGTGGATCTGGGACAGAATTCACTC
TCACCATCAGCAGCCTGCAGCCTGATGATTTTGCAACTTATTACT
GTCAACAATATAAGAGTCATTCTCCGCCCTTTGGCCAGGGGACC
AAGCTGGAGATCAAA

F6-113 VH

CAGGTACAGTTACAGCAGTGGGGCGCAGGACTGTTGAAGCCTT
CGGAGACCCTGTCCCTCACCTGCGCTGTCGATGGTGCGTCGGTC
AGTGATGACGACTGGACCTGGATCCGCCAGTCCCCAGGGAAGG
GCCTAGAGTGGATTGGACAAATCGATCAAACTGGAAGCTTCAA
TTACAACCCGTCCCTCAGGAGTCGAGTCACCATTTCACCAGAC
ACGTCCAAGAATCACTTTTCCCTGAGTCTGACTTCTGTGACCGC
CGCGGACACGGCTCTCTACTACTGTGCGAGAGGCGGACTGGTG
GTGACTGATGTCGGACAGGGTTGGGATGCTTTTGATGTCTGGGG
CCAAGGGACAATGGTCACCGTCTCTTCA

VL

GACATCCAGCTGACCCAGTCTCCATCTTCCGTGTCTGCATCTGT
GGGAGACAGAATCACCATCACTTGTCGGGCGAGTCAGGATATT
GACAACTGGTTAGCCTGGTATCAGCAGAAACCAGGGGAAGCCC
CTAAACTCCTGATCTATGCTGCATCCACTTTGCAAAGTGGGGTC
CCATCAAGGTTCAGCGGCAGTGGAGCTGGGACAGAATTCACTC
TCACCATCACCAGCCTGCAGCCTGAAGATTTTGCAGGTTACTTT
TGTCAACAGGGTGACAGTTTCCCGCTCACTTTCGGCGGGGGGA
CCAAGGTGGACATCAAA

F6-125 VH

CAGCTTCAGCTGGTACAGTCTGGAACGGAGGTGAAGAAGCCTG
GGGCCTCAGTGAAGGTCTCCTGCAAGGCTTCTGGTTACACCTTT
AGTGACAATGCTATCAACTGGGTGCGACAGGCCCCTGGACAAG

(continued)

| | |
|---|---|
| | GGCTTGAGTGGTTGGGATGGATCACCGCCAACAACGGAAACAC AAACTATGCACAGAAGATCCAGGGCAGAGTCACCATGACCACA GACACATCCACGAGCACGGTCTACATGGAGCTGAGGAGCCTGG GCTCTGACACACGGCCGTTTATTACTGTGCGAGGGAAGTGGT CAACTATGGTTTGGGGAGTCTACATTTTGACTATTGGGGCCAGG GAACCCTGGTCACCGTCTCCTCA |
| VL | TCCTACGAGCTGACTCAGCCACCCTCAGTGTCCGTGTCCCCAG GACAGACAGTCAGCATCAGCTGCTCTGGAGATAAACTGGGGAA TGACTATGCTAGTTGGTATCAGCAGAAGCCAGGCCAGTCCCCTC TGTTGGTCATTTATGAAGATGCCAAGCGGCCCTCAGGGATCCCT GAGCGATTCTCTGGCTCCAACTCCGGGAACACAGCCACTCTGA CCATCAGCGGGACCCAGGCTATGGATGACGCTGACTATTACTGT CAGGCGTGGGACGGCACCACTGTGATTTTCGGCGGAGGGACCA AGCTGACCGTCCTA |

## Claims

1. An antibody or an antigen-binding fragment thereof that specifically binds to RSV, comprising:

(1) a CDR-H1 set forth in SEQ ID NO: 1, a CDR-H2 set forth in SEQ ID NO: 2, a CDR-H3 set forth in SEQ ID NO: 3, a CDR-L1 set forth in SEQ ID NO: 4, a CDR-L2 set forth in SEQ ID NO: 5, and a CDR-L3 set forth in SEQ ID NO: 6;
(2) a CDR-H1 set forth in SEQ ID NO: 9, a CDR-H2 set forth in SEQ ID NO: 10, a CDR-H3 set forth in SEQ ID NO: 11, a CDR-L1 set forth in SEQ ID NO: 12, a CDR-L2 set forth in SEQ ID NO: 13, and a CDR-L3 set forth in SEQ ID NO: 14;
(3) a CDR-H1 set forth in SEQ ID NO: 17, a CDR-H2 set forth in SEQ ID NO: 18, a CDR-H3 set forth in SEQ ID NO: 19, a CDR-L1 set forth in SEQ ID NO: 20, a CDR-L2 set forth in SEQ ID NO: 21, and a CDR-L3 set forth in SEQ ID NO: 22; or
(4) a CDR-H1 set forth in SEQ ID NO: 25, a CDR-H2 set forth in SEQ ID NO: 26, a CDR-H3 set forth in SEQ ID NO: 27, a CDR-L1 set forth in SEQ ID NO: 28, a CDR-L2 set forth in SEQ ID NO: 29, and a CDR-L3 set forth in SEQ ID NO: 30.

2. The antibody or the antigen-binding fragment thereof according to claim 1, comprising:

(1) a VH set forth in SEQ ID NO: 7 and a VL set forth in SEQ ID NO: 8;
(2) a VH set forth in SEQ ID NO: 15 and a VL set forth in SEQ ID NO: 16;
(3) a VH set forth in SEQ ID NO: 23 and a VL set forth in SEQ ID NO: 24; or
(4) a VH set forth in SEQ ID NO: 31 and a VL set forth in SEQ ID NO: 32.

3. The antibody or the antigen-binding fragment thereof according to claim 1, comprising:

(1) a heavy chain constant region set forth in SEQ ID NO: 35, and
(2) a light chain constant region set forth in SEQ ID NO: 33 or 34.

4. The antibody or the antigen-binding fragment thereof according to claim 1, comprising an α heavy chain, a δ heavy chain, an ε heavy chain, a γ heavy chain or a μ heavy chain.

5. The antibody or the antigen-binding fragment thereof according to claim 1, belonging to an IgG1, IgG2, IgG3 or IgG4 subclass.

6. The antibody or the antigen-binding fragment thereof according to claim 1, comprising a λ light chain or a κ light chain.

7. The antibody or the antigen-binding fragment thereof according to claim 1, being a full-length antibody.

8. The antibody or the antigen-binding fragment thereof according to claim 1, being an antibody fragment selected from Fv, scFv, Fab, Fab', F(ab')$_2$ and xFab.

9. The antibody or the antigen-binding fragment thereof according to any one of claims 1-8, having one or more of properties of:

(1) being capable of specifically binding to an RSV A2 pre-F protein;
(2) being capable of specifically binding to an RSV A2 pre-F protein with an EC50 value less than 60, 55, 50, 45, 40, 35, 34, 33, 32, 31, 30, 29, 28, 27, 26, 25, 24, 23, 22, 21, 20, 19, 18, 17, 16, 15, 14, 13, 12, 11 or 10 ng/mL, or with an EC50 value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that of MEDI8897;
(3) being capable of specifically binding to an RSV A2 pre-F protein with a KD value less than 5.0, 1.0, 0.9, 0.8, 0.7, 0.6, 0.5, 0.4, 0.3, 0.2, 0.1, 0.09, 0.08, 0.07, 0.06, 0.05, 0.04, 0.03, 0.02, 0.01, 0.009, 0.008, 0.007, 0.006, 0.005, 0.004, 0.003, 0.002 or 0.001 nM, or with a KD value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that of MEDI8897;
(4) being capable of inhibiting RSV (e.g., subgroup A and/or subgroup B) from infecting a host cell;
(5) being capable of inhibiting an RSV subgroup A strain (e.g., A2) from infecting a host cell with an IC50 value less than 100, 90, 80, 70, 60, 50, 45, 40, 35, 30, 25, 20, 15, 10, 9, 8, 7, 6, 5, 4, 3, 2, 1 or 0.5 ng/mL, or with an IC50 value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that MEDI8897;
(6) being capable of inhibiting an RSV subgroup B strain (e.g., B9320) from infecting a host cell with an IC50 value less than 160, 150, 140, 130, 120, 110, 100, 90, 80, 70, 60, 50, 40, 30, 20, 15, 10, 9, 8, 7, 6, 5, 4 or 3 ng/mL, or with an IC50 value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that of MEDI8897;
(7) being capable of inhibiting an RSV subgroup B strain (e.g., B18537) from infecting a host cell with an IC50 value less than 50, 45, 40, 35, 30, 20, 15, 10, 9, 8, 7, 6, 5, 4, 3, 2 or 1 ng/mL, or with an IC50 value which is (e.g., 1% to 99%, e.g., at least 5%, 10%, 15%, 20%, 25%, 30%, 35%, 40%, 45%, 50%, 55%, 60%, 65%, 70%, 75%, 80%, 85%, 90%, 95% or 100%) less than that of MEDI8897;
(8) being capable of competing for or inhibiting the binding of MEDI8897 to an RSV A2 pre-F protein;
(9) being capable of inhibiting the binding of MEDI8897 to an RSV A2 pre-F protein by 1% to 100%, e.g., at least 5%, 10%, 15%, 20%, 25%, 30%, 35%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100%;
(10) being capable of competing for or inhibiting the binding of MK-1654 to an RSV A2 pre-F protein;
(11) being capable of inhibiting the binding of MK-1654 to an RSV A2 pre-F protein by 1% to 100%, e.g., at least 5%, 10%, 15%, 20%, 25%, 30%, 35%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100%;
(12) being capable of competing for or inhibiting the binding of Motavizumab to an RSV A2 pre-F protein;
(13) being capable of inhibiting the binding of Motavizumab to an RSV A2 pre-F protein by 1% to 100%, e.g., at least 5%, 10%, 15%, 20%, 25%, 30%, 35%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100%;
(14) being capable of competing for or inhibiting the binding of MPE8 to an RSV A2 pre-F protein;
(15) being capable of inhibiting the binding of MPE8 to an RSV A2 pre-F protein by 1% to 100%, e.g., at least 5%, 10%, 15%, 20%, 25%, 30%, 35%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100%;
(16) being capable of binding to an epitope Ø of an RSV A2 pre-F protein;
(17) being capable of binding to an epitope IV of an RSV A2 pre-F protein;
(18) being capable of binding to epitopes Ø and IV of an RSV A2 pre-F protein;
(19) being capable of binding to an epitope II of an RSV A2 pre-F protein;
(20) being capable of binding to an epitope III of an RSV A2 pre-F protein; and/or
(21) being capable of binding to epitopes II and III of an RSV A2 pre-F protein.

10. A polynucleotide encoding the antibody or the antigen-binding fragment thereof according to any one of claims 1-9.

11. The polynucleotide according to claim 10, comprising:

    (1) SEQ ID NOs: 36 and 37;
    (2) SEQ ID NOs: 38 and 39;
    (3) SEQ ID NOs: 40 and 41; or
    (4) SEQ ID NOs: 42 and 43.

12. A vector comprising the polynucleotide according to claim 10 or 11.

13. A host cell comprising the polynucleotide according to claim 10 or 11 or the vector according to claim 12.

14. The host cell according to claim 13, being a eukaryotic cell.

15. The host cell according to claim 14, being a CHO cell.

16. A method for generating an antibody or an antigen-binding fragment thereof, comprising:

    (a) culturing the host cell according to any one of claims 13-15 under conditions suitable for expression of the antibody or the antigen-binding fragment thereof, and
    (b) optionally, recovering the antibody or the antigen-binding fragment thereof.

17. A composition comprising the antibody or the antigen-binding fragment thereof according to any one of claims 1-9.

18. The antibody or the antigen-binding fragment thereof according to any one of claims 1-9 or the composition according to claim 17, for use as a medicament.

19. The antibody or the antigen-binding fragment thereof according to any one of claims 1-9 or the composition according to claim 17, for use in the treatment of a disease.

20. The antibody or the antigen-binding fragment thereof or the composition according to claim 19, wherein the disease is a lower respiratory tract infection.

21. Use of the antibody or the antigen-binding fragment thereof according to any one of claims 1-9 or the composition according to claim 17 for the manufacture of a medicament for treating a disease.

22. The use according to claim 21, wherein the disease is a lower respiratory tract infection.

23. A method for treating a disease in an individual, comprising administering to the individual a therapeutically effective amount of the antibody or the antigen-binding fragment thereof according to any one of claims 1-9 or the composition according to claim 17.

24. The method according to claim 23, wherein the disease is a lower respiratory tract infection.

25. The invention as described in the description.

FIG. 1-1

FIG. 1-2

FIG. 2-1

FIG. 2-2

**RSV A2**

**RSV B9320**

**RSV B18537**

Legend:
- F6-10
- F6-113
- F6-125
- MEDI8897

FIG. 3-1

FIG. 3-2

FIG. 4

## INTERNATIONAL SEARCH REPORT

| International application No. |
|---|
| **PCT/CN2023/137388** |

### A. CLASSIFICATION OF SUBJECT MATTER

C07K16/10(2006.01)i; C12N15/09(2006.01)i; C12N5/10(2006.01)i; C12N1/19(2006.01)i; C12N1/21(2006.01)i; C12N1/15(2006.01)i; A61K39/395(2006.01)i; A61P31/14(2006.01)i; A61P11/00(2006.01)i

According to International Patent Classification (IPC) or to both national classification and IPC

### B. FIELDS SEARCHED

Minimum documentation searched (classification system followed by classification symbols)

IPC: C07K C12N A61K A61P

Documentation searched other than minimum documentation to the extent that such documents are included in the fields searched

Electronic data base consulted during the international search (name of data base and, where practicable, search terms used)

CNABS, CNKI, CNTXT, DWPI, SIPOABS, EPTXT, USTXT, WOTXT, JPTXT, NCBI, 中国专利生物序列检索系统, China Patent Biological Sequence Search System, Genbank, EMBL, STN and search terms: 吸道合胞病毒, Respiratory Syncytial Virus, RSV, 抗体, antibody, 结合, binding, SEQ ID Nos: 1-43

### C. DOCUMENTS CONSIDERED TO BE RELEVANT

| Category* | Citation of document, with indication, where appropriate, of the relevant passages | Relevant to claim No. |
|---|---|---|
| A | CN 103998465 A (PONTIFICIA UNIVERSIDAD CATOLICA DE CHILE) 20 August 2014 (2014-08-20) claims, and embodiments | 1-22 |
| A | CN 108431036 A (MERCK SHARP & DOHME CORP.) 21 August 2018 (2018-08-21) claims, and embodiments | 1-22 |
| A | CN 110418803 A (MEDIMMUNE LIMITED) 05 November 2019 (2019-11-05) claims, and embodiments | 1-22 |
| A | CN 113249333 A (GUIZHOU PROVINCIAL PEOPLE'S HOSPITAL) 13 August 2021 (2021-08-13) claims, and embodiments | 1-22 |
| A | US 2002141990 A1 (SMITHKLINE BEECHAM CORP.) 03 October 2002 (2002-10-03) claims, and embodiments | 1-22 |
| A | US 2005175986 A1 (SMITHKLINE BEECHAM PLC) 11 August 2005 (2005-08-11) claims, and embodiments | 1-22 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **07 February 2024** | **28 February 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/137388**

**C.     DOCUMENTS CONSIDERED TO BE RELEVANT**

| Category* | Citation of document, with indication, where appropriate, of the relevant passages | Relevant to claim No. |
|---|---|---|
| A | US 2006099220 A1 (MEDIMMUNE INC.) 11 May 2006 (2006-05-11)<br>claims, and embodiments | 1-22 |

Form PCT/ISA/210 (second sheet) (July 2022)

## INTERNATIONAL SEARCH REPORT

| | International application No. |
|---|---|
| | **PCT/CN2023/137388** |

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2023/137388** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **23-24**
   because they relate to subject matter not required to be searched by this Authority, namely:

   Disease treatment methods (PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

46

**INTERNATIONAL SEARCH REPORT**
Information on patent family members

International application No.

**PCT/CN2023/137388**

| Patent document cited in search report | | | Publication date (day/month/year) | Patent family member(s) | | | Publication date (day/month/year) |
|---|---|---|---|---|---|---|---|
| CN | 103998465 | A | 20 August 2014 | IN | 4312014 | A | 18 September 2015 |
| | | | | AR | 088987 | A1 | 23 July 2014 |
| | | | | EP | 2784088 | A2 | 01 October 2014 |
| | | | | EP | 2784088 | A4 | 13 May 2015 |
| | | | | EP | 2784088 | B1 | 25 October 2017 |
| | | | | BR | 112014012608 | A2 | 27 October 2020 |
| | | | | WO | 2013076702 | A2 | 30 May 2013 |
| | | | | WO | 2013076702 | A3 | 25 July 2013 |
| | | | | CL | 2011003002 | A1 | 25 May 2012 |
| | | | | ZA | 201403564 | B | 26 August 2015 |
| | | | | US | 2014348858 | A1 | 27 November 2014 |
| | | | | US | 9273122 | B2 | 01 March 2016 |
| | | | | PE | 20141683 | A1 | 28 November 2014 |
| | | | | PT | 2784088 | T | 31 January 2018 |
| | | | | ES | 2657619 | T3 | 06 March 2018 |
| | | | | CA | 2856258 | A1 | 30 May 2013 |
| | | | | CA | 2856258 | C | 01 May 2018 |
| | | | | MX | 2014006248 | A | 03 March 2015 |
| | | | | MX | 349235 | B | 19 July 2017 |
| CN | 108431036 | A | 21 August 2018 | PE | 20181270 | A1 | 03 August 2018 |
| | | | | IL | 258743 | A | 28 June 2018 |
| | | | | IL | 258743 | B | 01 November 2022 |
| | | | | IL | 258743 | B2 | 01 March 2023 |
| | | | | TN | 2018000134 | A1 | 04 October 2019 |
| | | | | CL | 2018001141 | A1 | 12 October 2018 |
| | | | | WO | 2017075124 | A1 | 04 May 2017 |
| | | | | JO | 3555 | B1 | 05 July 2020 |
| | | | | MY | 192714 | A | 05 September 2022 |
| | | | | SG | 10201809694 | PA | 28 December 2018 |
| | | | | BR | 112018008577 | A2 | 30 October 2018 |
| | | | | BR | 112018008577 | A8 | 07 February 2023 |
| | | | | US | 2018215810 | A1 | 02 August 2018 |
| | | | | US | 10072072 | B2 | 11 September 2018 |
| | | | | US | 2018215809 | A1 | 02 August 2018 |
| | | | | US | 10323079 | B2 | 18 June 2019 |
| | | | | TW | 201722991 | A | 01 July 2017 |
| | | | | TWI | 743057 | B | 21 October 2021 |
| | | | | PH | 12018500916 | A1 | 05 November 2018 |
| | | | | JP | 2019503648 | A | 14 February 2019 |
| | | | | JP | 6880014 | B2 | 02 June 2021 |
| | | | | US | 2018215808 | A1 | 02 August 2018 |
| | | | | US | 10358480 | B2 | 23 July 2019 |
| | | | | US | 2020002407 | A1 | 02 January 2020 |
| | | | | US | 11008380 | B2 | 18 May 2021 |
| | | | | GEP | 20217260 | B | 25 May 2021 |
| | | | | EP | 3368562 | A1 | 05 September 2018 |
| | | | | AU | 2020200729 | A1 | 20 February 2020 |
| | | | | US | 2017121394 | A1 | 04 May 2017 |
| | | | | US | 9963500 | B2 | 08 May 2018 |
| | | | | ZA | 201802369 | B | 30 January 2019 |

Form PCT/ISA/210 (patent family annex) (July 2022)

## INTERNATIONAL SEARCH REPORT
### Information on patent family members

International application No.

**PCT/CN2023/137388**

| Patent document cited in search report | | | Publication date (day/month/year) | Patent family member(s) | | | Publication date (day/month/year) |
|---|---|---|---|---|---|---|---|
| | | | | KR | 20180069056 | A | 22 June 2018 |
| | | | | KR | 102140113 | B1 | 31 July 2020 |
| | | | | NI | 201800052 | A | 24 July 2018 |
| | | | | AU | 2022202475 | A1 | 05 May 2022 |
| | | | | MA | 43108 | A | 05 September 2018 |
| | | | | AU | 2016344070 | A1 | 10 May 2018 |
| | | | | AU | 2016344070 | B2 | 07 November 2019 |
| | | | | CO | 2018004321 | A2 | 22 November 2018 |
| | | | | HK | 1254950 | A1 | 02 August 2019 |
| | | | | US | 2023212269 | A1 | 06 July 2023 |
| | | | | JOP | 20200091 | A1 | 30 October 2022 |
| | | | | UA | 127516 | C2 | 20 September 2023 |
| | | | | SG | 11201803524 | UA | 30 May 2018 |
| | | | | US | 2021188951 | A1 | 24 June 2021 |
| | | | | US | 11566065 | B2 | 31 January 2023 |
| | | | | CA | 3001878 | A1 | 04 May 2017 |
| | | | | CA | 3001878 | C | 14 February 2023 |
| | | | | MX | 2018005047 | A | 06 September 2018 |
| | | | | SV | 2018005678 | A | 14 December 2018 |
| CN | 110418803 | A | 05 November 2019 | CL | 2019002478 | A1 | 24 January 2020 |
| | | | | CA | 3051864 | A1 | 07 September 2018 |
| | | | | MX | 2019010408 | A | 19 December 2019 |
| | | | | IL | 268202 | A | 26 September 2019 |
| | | | | GB | 201802974 | D0 | 11 April 2018 |
| | | | | GB | 2564175 | A | 09 January 2019 |
| | | | | GB | 2564175 | B | 08 December 2021 |
| | | | | TW | 202228779 | A | 01 August 2022 |
| | | | | CO | 2019009034 | A2 | 30 August 2019 |
| | | | | ECSP | 19062769 | A | 30 September 2019 |
| | | | | KR | 20190125318 | A | 06 November 2019 |
| | | | | KR | 102359181 | B1 | 08 February 2022 |
| | | | | PH | 12019501979 | A1 | 29 June 2020 |
| | | | | AU | 2018227502 | A1 | 15 August 2019 |
| | | | | AU | 2018227502 | B2 | 11 August 2022 |
| | | | | US | 2021317191 | A1 | 14 October 2021 |
| | | | | US | 11286294 | B2 | 29 March 2022 |
| | | | | EP | 3589648 | A1 | 08 January 2020 |
| | | | | EP | 3589648 | A4 | 23 December 2020 |
| | | | | US | 2023399383 | A1 | 14 December 2023 |
| | | | | BR | 112019017755 | A2 | 07 April 2020 |
| | | | | SG | 11201906853 | QA | 27 September 2019 |
| | | | | CR | 20190400 | A | 03 December 2019 |
| | | | | AU | 2022263564 | A1 | 15 December 2022 |
| | | | | US | 2020339667 | A1 | 29 October 2020 |
| | | | | US | 11667698 | B2 | 06 June 2023 |
| | | | | JP | 2022095697 | A | 28 June 2022 |
| | | | | TW | 201840334 | A | 16 November 2018 |
| | | | | TWI | 761453 | B | 21 April 2022 |
| | | | | EA | 201991701 | A1 | 04 March 2020 |
| | | | | AR | 111083 | A1 | 29 May 2019 |

Form PCT/ISA/210 (patent family annex) (July 2022)

# EP 4 588 936 A1

## INTERNATIONAL SEARCH REPORT
### Information on patent family members

International application No.

**PCT/CN2023/137388**

| Patent document cited in search report | | | Publication date (day/month/year) | Patent family member(s) | | | Publication date (day/month/year) |
|---|---|---|---|---|---|---|---|
| | | | | ZA | 202006797 | B | 29 March 2023 |
| | | | | KR | 20220020420 | A | 18 February 2022 |
| | | | | US | 2018251526 | A1 | 06 September 2018 |
| | | | | US | 10774133 | B2 | 15 September 2020 |
| | | | | JP | 2020509031 | A | 26 March 2020 |
| | | | | JP | 7046088 | B2 | 01 April 2022 |
| | | | | WO | 2018160722 | A1 | 07 September 2018 |
| CN | 113249333 | A | 13 August 2021 | None | | | |
| US | 2002141990 | A1 | 03 October 2002 | US | 2005019758 | A1 | 27 January 2005 |
| US | 2005175986 | A1 | 11 August 2005 | None | | | |
| US | 2006099220 | A1 | 11 May 2006 | JP | 2008513540 | A | 01 May 2008 |
| | | | | EP | 1793850 | A2 | 13 June 2007 |
| | | | | EP | 1793850 | A4 | 30 June 2010 |
| | | | | JP | 2011252023 | A | 15 December 2011 |
| | | | | EP | 3073267 | A1 | 28 September 2016 |
| | | | | US | 7700720 | B2 | 20 April 2010 |
| | | | | JP | 2016135133 | A | 28 July 2016 |
| | | | | JP | 6407187 | B2 | 17 October 2018 |
| | | | | CA | 2580921 | A1 | 30 March 2006 |
| | | | | CA | 2580921 | C | 12 April 2016 |
| | | | | WO | 2006034292 | A2 | 30 March 2006 |
| | | | | WO | 2006034292 | A3 | 16 April 2009 |
| | | | | US | 2010278852 | A1 | 04 November 2010 |
| | | | | US | 9096658 | B2 | 04 August 2015 |
| | | | | AU | 2005286770 | A1 | 30 March 2006 |
| | | | | JP | 2014193876 | A | 09 October 2014 |
| | | | | JP | 5992014 | B2 | 14 September 2016 |
| | | | | US | 2015328306 | A1 | 19 November 2015 |
| | | | | US | 9913894 | B2 | 13 March 2018 |

Form PCT/ISA/210 (patent family annex) (July 2022)

## REFERENCES CITED IN THE DESCRIPTION

### Patent documents cited in the description

- CN 202211571449
- CN 202310007283
- US 20040101920 A
- WO 2012130831 A1
- US 5869046 A
- EP 404097 A
- WO 199301161 A
- WO 9316185 A
- US 5571894 A
- US 5587458 A
- US 6248516 B1
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 7985840 B
- US 7785903 B
- US 8679490 B
- US 20050079574 A
- US 20070117126 A
- US 20070237764 A
- US 20070292936 A
- US 5731168 A
- WO 2009089004 A
- US 4676980 A
- WO 2011034605 A
- WO 9850431 A
- WO 200177342 A
- WO 2008024715 A
- WO 2010115589 A
- WO 2010112193 A
- WO 2010136172 A
- WO 2010145792 A
- WO 2013026831 A
- US 20080069820 A
- WO 2015095539 A
- WO 2009080252 A
- WO 2015150447 A
- WO 2009080253 A
- WO 2009080251 A
- WO 2016016299 A
- WO 2016172485 A
- WO 2006082515 A
- US 20030157108 A
- US 20040093621 A
- WO 2004056312 A
- WO 2003085107 A
- US 2004259150 A
- US 2005031613 A
- US 2004132140 A
- US 2004110282 A
- WO 9954342 A
- WO 2004065540 A
- WO 2003011878 A
- WO 199730087 A
- WO 199858964 A
- WO 199922764 A
- US 5500362 A
- WO 2006029879 A
- WO 2005100402 A
- WO 2013120929 A1
- US 6737056 B
- US 7332581 B
- US 6194551 B
- WO 9951642 A
- US 20050014934 A1, Hinton
- US 7371826 B
- WO 2014177460 A1
- US 5648260 A
- US 5624821 A
- WO 9429351 A
- US 5648237 A
- US 5789199 A
- US 5840523 A
- US 5959177 A
- US 6040498 A
- US 6420548 B
- US 7125978 B
- US 6417429 B
- US 4737456 A
- US 20050260186 A
- US 20060104968 A
- US 6267958 B
- US 6171586 B
- WO 2006044908 A

**Non-patent literature cited in the description**

- *Meth. Mol. Biol.*, 2004, vol. 248, 443-463
- *Prot. Sci.*, 2000, vol. 9, 487-496
- **HARLOW** ; **LANE**. Antibodies. Cold Spring Harbor Press
- **JUNGHANS et al.** *Cancer Res.*, 1990, vol. 50, 1495-1502
- **KABAT et al.** Sequences of Proteins of Immunological Interest. Public Health Service, National Institutes of Health, 1991
- **KABAT et al.** Sequences of Proteins of Immunological Interest. NIH Publication 91-3242, 1991, vol. 1-3
- **CHOTHIA** ; **LESK**. *J. Mol. Biol.*, 1987, vol. 196, 901-917
- **MACCALLUM et al.** *J. Mol. Biol.*, 1996, vol. 262, 732-745
- **FLATMAN et al.** *J. Chromatogr., B*, 2007, vol. 848, 79-87
- **W. R. PEARSON** ; **D. J. LIPMAN**. Improved Tools for Biological Sequence Analysis. *PNAS*, 1988, vol. 85, 2444-2448
- **W. R. PEARSON**. Effective protein sequence comparison. *Meth. Enzymol.*, 1996, vol. 266, 227-258
- **PEARSON et al.** *Genomics*, 1997, vol. 46, 24-36
- **KINDT et al.** Kuby Immunology. W.H. Freeman and Co, 2007, 91
- **PORTOLANO et al.** *J. Immunol.*, 1993, vol. 150, 880-887
- **CLARKSON et al.** *Nature*, 1991, vol. 352, 624-628
- **CHEN et al.** *J. Mol. Biol.*, 1999, vol. 293, 865-881
- **PRESTA et al.** *Cancer Res.*, 1997, vol. 57, 4593-4599
- **HOLLIGER** ; **HUDSON**. *Nature Biotechnology*, 2005, vol. 23, 1126-1136
- **HUDSON et al.** *Nat. Med.*, 2003, vol. 9, 129-134
- **HOLLINGER et al.** *Proc. Natl. Acad. Sci. USA*, 1993, vol. 90, 6444-6448
- **PLUCKTHÜN**. The Pharmacology of Monoclonal Antibodies. Springer-Verlag, 1994, vol. 113, 269-315
- **MORRISON et al.** *Proc. Natl. Acad. Sci. USA*, 1984, vol. 81, 6851-6855
- **ALMAGRO** ; **FRANSSON**. *Front. Biosci.*, 2008, vol. 13, 1619-1633
- **RIECHMANN et al.** *Nature*, 1988, vol. 332, 323-329
- **QUEEN et al.** *Proc. Nat'l Acad. Sci. USA*, 1989, vol. 86, 10029-10033
- **KASHMIRI et al.** *Methods*, 2005, vol. 36, 25-34
- **PADLAN**. *Mol. Immunol.*, 1991, vol. 28, 489-498
- **DALL'ACQUA et al.** *Methods*, 2005, vol. 36, 43-60
- **OSBOURN et al.** *Methods*, 2005, vol. 36, 61-68
- **KLIMKA et al.** *Br. J. Cancer*, 2000, vol. 83, 252-260
- **SIMS et al.** *J. Immunol.*, 1993, vol. 151, 2296
- **CARTER et al.** *Proc. Natl. Acad. Sci. USA*, 1992, vol. 89, 4285
- **PRESTA et al.** *J. Immunol.*, 1993, vol. 151, 2623
- **BACA et al.** *J. Biol. Chem.*, 1997, vol. 272, 10678-10684
- **ROSOK et al.** *J. Biol. Chem.*, 1996, vol. 271, 22611-22618
- **VAN DIJK** ; **VAN DE WINKEL**. *Curr. Opin. Pharmacol.*, 2001, vol. 5, 368-74
- **LONBERG**. *Curr. Opin. Immunol.*, 2008, vol. 20, 450-459
- **LONBERG**. *Nat. Biotech.*, 2005, vol. 23, 1117-1125
- **KOZBOR**. *J. Immunol.*, 1984, vol. 133, 3001
- **BRODEUR et al.** Monoclonal Antibody Production Techniques and Applications. Marcel Dekker, Inc, 1987, 51-63
- **BOERNER et al.** *J. Immunol.*, 1991, vol. 147, 86
- **LI et al.** *Proc. Natl. Acad. Sci. USA*, 2006, vol. 103, 3557-3562
- **NI**. *Xiandai Mianyixue*, 2006, vol. 26 (4), 265-268
- **VOLLMERS** ; **BRANDLEIN**. *Histology and Histopathology*, 2005, vol. 20 (3), 927-937
- **VOLLMERS** ; **BRANDLEIN**. *Methods and Findings in Experimental and Clinical Pharmacology*, 2005, vol. 27 (3), 185-91
- **LERNER et al.** *Nature*, 2016, vol. 16, 498-508
- **FRENZEL et al.** *mAbs*, 2016, vol. 8, 1177-1194
- **BAZAN et al.** *Human Vaccines and Immunotherapeutics*, 2012, vol. 8, 1817-1828
- **ZHAO et al.** *Critical Reviews in Biotechnology*, 2016, vol. 36, 276-289
- **HOOGENBOOM et al.** Methods in Molecular Biology. Human Press, 2001, vol. 178, 1-37
- **MARKS** ; **BRADBURY**. Methods in Molecular Biology. Human Press, 2003, vol. 248, 161-175
- **WINTER et al.** *Annual Review of Immunology*, 1994, vol. 12, 433-455
- **GRIFFITHS et al.** *EMBO Journal*, 1993, vol. 12, 725-734
- **HOOGENBOOM** ; **WINTER**. *Journal of Molecular Biology*, 1992, vol. 227, 381-388

- **SCHOLLER et al.** *Methods in Molecular Biology*, 2012, vol. 503, 135-56
- **CHERF et al.** *Methods in Molecular biology*, 2015, vol. 1319, 155-175
- **ZHAO et al.** *Methods in Molecular Biology*, 2012, vol. 889, 73-84
- **HE et al.** *Nucleic Acids Research*, 1997, vol. 25, 5132-5134
- **HANES et al.** *PNAS*, 1997, vol. 94, 4937-4942
- **MILSTEIN ; CUELLO.** *Nature*, 1983, vol. 305, 537
- **ATWELL et al.** *J. Mol. Biol.*, 1997, vol. 270, 26
- **BRENNAN et al.** *Science*, 1985, vol. 229, 81
- **KOSTELNY et al.** *J. Immunol.*, 1992, vol. 148 (5), 1547-1553
- **GRUBER et al.** *J. Immunol.*, 1994, vol. 152, 5368
- **TUTT et al.** *J. Immunol.*, 1991, vol. 147, 60
- **SCHAEFER et al.** *PNAS*, 2011, vol. 108, 1187-1191
- **KLEIN et al.** *MAbs*, 2016, vol. 8, 1010-20
- **SPIESS et al.** *Mol Immunol*, 2015, vol. 67, 95-106
- **CHOWDHURY.** *Methods Mol. Biol.*, 2008, vol. 207, 179-196
- **CUNNINGHAM ; WELLS.** *Science*, 1989, vol. 244, 1081-1085
- **WRIGHT et al.** *TIBTECH*, 1997, vol. 15, 26-32
- **RIPKA et al.** *Arch. Biochem. Biophys.*, 1986, vol. 249, 533-545
- **YAMANE-OHNUKI et al.** *Biotech. Bioeng.*, 2004, vol. 87, 614-622
- **KANDA, Y. et al.** *Biotechnol. Bioeng.*, 2006, vol. 94 (4), 680-688
- **UMANA et al.** *Nat Biotechnol*, 1999, vol. 17, 176-180
- **FERRARA et al.** *Biotechn Bioeng*, 2006, vol. 93, 851-861
- **RAVETCH ; KINET.** *Annu. Rev. Immunol.*, 1991, vol. 9, 457-492
- **HELLSTROM, I. et al.** *Proc. Nat'l Acad. Sci. USA*, 1986, vol. 83, 7059-7063
- **HELLSTROM, I. et al.** *Proc. Nat'l Acad. Sci. USA*, 1985, vol. 82, 1499-1502
- **BRUGGEMANN, M. et al.** *J. Exp. Med.*, 1987, vol. 166, 1351-1361
- **CLYNES et al.** *Proc. Nat'l Acad. Sci. USA*, 1998, vol. 95, 652-656
- **GAZZANO-SANTORO et al.** *J. Immunol. Methods*, 1996, vol. 202, 163
- **CRAGG, M.S. et al.** *Blood*, 2003, vol. 101, 1045-1052
- **CRAGG, M.S. ; M.J. GLENNIE.** *Blood*, 2004, vol. 103, 2738-2743
- **PETKOVA, S.B et al.** *Int'l. Immunol.*, 2006, vol. 18 (12), 1759-1769
- **SHIELDS et al.** *J. Biol. Chem.*, 2001, vol. 9 (2), 6591-6604
- **IDUSOGIE et al.** *J. Immunol.*, 2000, vol. 164, 4178-4184
- **GUYER et al.** *J. Immunol.*, 1976, vol. 117 (587)
- **KIM et al.** *J. Immunol.*, 1994, vol. 24, 249
- **DALL'ACQUA, W. F et al.** *J. Biol. Chem.*, 2006, vol. 281, 23514-23524
- **DALL'ACQUA, W. F. et al.** *J. Immunol*, 2002, vol. 169, 5171-5180
- **MEDESAN, C. et al.** *Eur. J. Immunol.*, 1996, vol. 26, 2533
- **FIRAN, M. et al.** *Int. Immunol.*, 2001, vol. 13, 993
- **KIM, J. K. et al.** *Eur. J. Immunol.*, 1994, vol. 24, 542
- **KIM, J. K. et al.** *Eur. J. Immunol.*, 1999, vol. 29, 2819
- **SHIELDS, R.L. et al.** *J. Biol. Chem.*, 2001, vol. 276, 6591-6604
- **YEUNG, Y. A. et al.** *J. Immunol.*, 2009, vol. 182, 7667-7671
- **GREVYS, A. et al.** *J. Immunol.*, 2015, vol. 194, 5497-5508
- **DUNCAN ; WINTER.** *Nature*, 1988, vol. 322, 738-40
- **SCHAEFER, W. et al.** *PNAS*, 2011, vol. 108, 11187-1191
- **CARTER, P. et al.** *Immunotechnol*, 1996, vol. 2, 73
- **CHARLTON, K. A.** Methods in Molecular Biology. Humana Press, 2003, vol. 248, 245-254
- **GERNGROSS, T.U.** *Nat. Biotech.*, 2004, vol. 22, 1409-1414
- **LI et al.** *Nat. Biotech.*, 2006, vol. 24, 210-215
- **GRAHAM, F. L. et al.** *J. Gen Virol.*, 1977, vol. 36, 59-74
- **MATHER, J. P.** *Biol. Reprod.*, 1980, vol. 23, 243-252
- **MATHER, J. P et al.** *Annals N. Y. Acad. Sci.*, 1982, vol. 383, 44-68
- **URLAUB, G. et al.** *Proc. Natl. Acad. Sci. USA*, 1980, vol. 77, 4216-4220
- **YAZAKI, P. ; WU, A.M.** Methods in Molecular Biology. Humana Press, 2004, vol. 248, 255-268
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- Remington's Pharmaceutical Sciences. 1980